# EUROPEAN PATENT APPLICATION

(11) **EP 4 094 777 A1**
(43) Date of publication of application: **30.11.2022**
(21) Application number: 21744016.3
(22) Date of filing: 19.01.2021
(51) Int. Cl.: A61K 39/00, A61P 37/00, C07K 16/00

(54) **RECOMBINANT FULLY HUMAN ANTI-TIGIT MONOCLONAL ANTIBODY PREPARATIONS, PREPARATION METHOD THEREFOR AND USE THEREOF**

(30) Priority: 21.01.2020 CN 202010070349
(71) Applicant: Innovent Biologics (Suzhou) Co., Ltd., Suzhou, Jiangsu 215123 (CN)
(72) Inventor: ZHANG, Haitao, Suzhou, Jiangsu 215123 (CN); MA, Liqiang, Suzhou, Jiangsu 215123 (CN); WANG, Yinjue, Suzhou, Jiangsu 215123 (CN)
(74) Representative: Ruscoe, Peter James
(86) International application number: PCT/CN2021/072690
(87) International publication number: WO 2021/147854

(57) **Abstract**

The present invention relates to formulations comprising an anti-TIGIT antibody, and in particular to a pharmaceutical formulation comprising an anti-TIGIT antibody, a buffer, a stabilizer and a surfactant. Furthermore, the present invention further relates to therapeutic or prophylactic use of these formulations.

## Description

### TECHNICAL FIELD

The present invention relates to the field of antibody formulations. Particularly, the present invention relates to a pharmaceutical formulation, more particularly a stable liquid formulation, comprising a recombinant fully human antibodies against T-cell immunoreceptor with immunoglobulin and immunoreceptor tyrosine-based inhibitory motif domains (also known as anti-TIGIT antibody), a method for preparing the pharmaceutical formulation, and therapeutic and/or prophylactic use of the pharmaceutical formulation.

### BACKGROUND

The stability of a drug is one of key indexes for ensuring the efficacy and safety. A good formulation is a key prerequisite to keep the efficacy and safety of a drug over the shelf life. However, due to the complexity of antibodies and their metabolism pathway, it is currently impossible to predict the formulation conditions required to optimize antibody stability. This is particularly essential considering that different antibodies generally have different CDR sequences, and that such sequence differences will result in different stability properties of the antibodies in a solution. Therefore, based on the stringent requirements for safety and efficacy of antibodies for human use, it is necessary to optimize the formulation for each antibody.

TIGIT (T-cell immunoreceptor with immunoglobulin and immunoreceptor tyrosine-based inhibitory motif domains, also known as WUCAM, Vstm3, or Vsig9) is originally discovered as a member of the CD28 family by comparison in bioinformatics. An antibody binding to human TIGIT has been demonstrated to be useful for treating cancers. See, for example, WO2006/124667. Antibody blockade of PD-L1 and TIGIT can increase the CD8⁺ T cell-mediated tumor rejection in a synergistic manner in a mouse model. Grogan et al. (2014) J. Immunol. 192(1) Suppl. 203.15; Johnston et al. (2014) Cancer Cell 26:1-15. Similar results were obtained in an animal model of melanoma. Inozume et al. (2014) J. Invest. Dermatol. 134:S121 - Abstract 693. Anti-TIGIT antibodies that bind with high specificity to TIGIT have been described, for example, in PCT Application No. PCT/CN2019/097665.

Although some anti-TIGIT antibody formulations have been proposed, there's still a need in the art for new pharmaceutical formulations comprising anti-TIGIT antibodies that are sufficiently stable and suitable for administration to human subjects. Furthermore, for such antibody formulations, the simplicity of formulation and ease of use may also be advantageous.

### SUMMARY

The present invention satisfies the above-described need by providing a pharmaceutical formulation comprising an antibody specifically binding to TIGIT. The antibody formulation disclosed herein exhibits excellent stability against a variety of stability-influencing factors, such as temperature, repeated freezing-thawing, and shaking.

As such, in one aspect, the present invention provides a liquid antibody formulation comprising: (i) an anti-TIGIT antibody, (ii) a buffer, (iii) a stabilizer, and (iv) a surfactant.

In one embodiment, the anti-TIGIT antibody comprises a heavy chain variable region VH and a light chain variable region VL, wherein the heavy chain variable region comprises a sequence of SEQ ID NO: 7 or a sequence having at least 90% identity thereto, and the light chain variable region comprises a sequence of SEQ ID NO: 8 or a sequence having at least 90% identity thereto:

QVQLVQSGAEVKKPGASVKVSCKASGYTFTEYYMHWVRQAPGQGLEWMGIISPSAGSTKYAQKFQGR
VTMTRDTSTSTVYMELSSLRSEDTAVYYCARDHDIRLAGRLADYWGQGTLVTVSS (SEQ ID NO: 7);
DIQMTQSPSSVSASVGDRVTITCRASQGISSWLAWYQQKPGKAPKLLISAASSLQSGVPSRFSGSGSGTDF
TLTISSLQPEDFATYYCQQAVILPITFGGGTKVEIK (SEQ ID NO: 8).

In one embodiment, the anti-TIGIT antibody comprises:
- a heavy chain VH CDR1 of YTFTEYYMH (SEQ ID NO: 1);
- a heavy chain VH CDR2 of IISPSAGSTKYAQKFQG (SEQ ID NO: 2);
- a heavy chain VH CDR3 of ARDHDIRLAGRLADY (SEQ ID NO: 3);
- a light chain VL CDR1 of RASQGISSWLA (SEQ ID NO: 4);
- a light chain VL CDR2 of AASSLQS (SEQ ID NO: 5); and
- a light chain VL CDR3 of QQAVILPIT (SEQ ID NO: 6).

In one embodiment, the anti-TIGIT antibody is an IgG4 antibody comprising a heavy chain and a light chain, wherein the heavy chain comprises a sequence of SEQ ID NO: 9 or a sequence having at least 90% identity thereto, and the light chain comprises a sequence of SEQ ID NO: 10 or a sequence having at least 90% identity thereto:

QVQLVQSGAEVKKPGASVKVSCKASGYTFTEYYMHWVRQAPGQGLEWMGIISPSAGSTKYAQKF
QGRVTMTRDTSTSTVYMELSSLRSEDTAVYYCARDHDIRLAGRLADYWGQGTLVTVSSASTKGPSV
FPLAPCSRSTSESTAALGCLVKDYFPEPVTVSWNSGALTSGVHTFPAVLQSSGLYSLSSVVTVPSSSLGTK
TYTCNVDHKPSNTKVDKRVESKYGPPCPPCPAPEAAGGPSVFLFPPKPKDTLMISRTPEVTCVVVDVSQE
DPEVQFNWYVDGVEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYKTTPPVLDSDGSFFLY
SRLTVDKSRWQEGNVFSCSVMHEALHNHYTQKSLSLSLG (SEQ ID NO: 9)

DIQMTQSPSSVSASVGDRVTITCRASQGISSWLAWYQQKPGKAPKLLISAASSLQSGVPSRFSGSGSG
TDFTLTISSLQPEDFATYYCQQAVILPITFGGGTKVEIKRTVAAPSVFIFPPSDEQLKSGTASVVCLLNN
FYPREAKVQWKVDNALQSGNSQESVTEQDSKDSTYSLSSTLTLSKADYEKHKVYACEVTHQGLSSPVTK
SFNRGEC (SEQ ID NO: 10).

Preferably, the anti-TIGIT antibody is the anti-TIGIT monoclonal antibody ADI-30278 disclosed in PCT Application No. PCT/CN2019/097665 (International Application Date: Jul. 25, 2019), consisting of the heavy chain sequence of SEQ ID NO: 9 and the light chain sequence of SEQ ID NO: 10.

In one embodiment, the anti-TIGIT antibody is an anti-TIGIT antibody recombinantly expressed in 293 cells or CHO cells.

In one embodiment, the anti-TIGIT antibody in the liquid antibody formulation disclosed herein has a concentration of about 1-150 mg/mL. In another embodiment, the anti-TIGIT antibody in the liquid antibody formulation disclosed herein has a concentration of about 10-100 mg/mL, preferably 20-60 mg/mL, particularly about 50 mg/mL. In other embodiments, the anti-TIGIT antibody in the liquid antibody formulation disclosed herein has a concentration of about 10, 15, 20, 25, 30, 35, 40, 50, 60, 70, 80, 90 or 100 mg/mL.

In one embodiment, the buffer in the liquid antibody formulation disclosed herein has a concentration of about 5-50 mM. In one embodiment, the buffer in the liquid antibody formulation disclosed herein has a concentration of about 5-30 mM, e.g., about 5, 10, 15, 20, 25 or 30 mM. In one embodiment, the buffer is a histidine buffer, preferably, the buffer consists of histidine and histidine hydrochloride. In one preferred embodiment, the buffer is a histidine buffer at about 5-30 mM, for example, 10-20 mM, e.g., histidine at about 10 mM.

In one embodiment, the stabilizer in the liquid antibody formulation disclosed herein has a concentration of about 50-500 mM. In one embodiment, the stabilizer in the liquid antibody formulation disclosed herein has a concentration of about 100-400 mM, e.g., about 100, 150, 200, 250, 300, 350 or 400 mM.

In one embodiment, the stabilizer is selected from polyols (e.g., sorbitol), saccharides (e.g., sucrose), amino acids (e.g., arginine or arginine hydrochloride) and any combination thereof. In one embodiment, the stabilizer comprises sorbitol at about 20-80 mg/mL, e.g., sorbitol at 20, 25, 30, 35, 40, 45, 50, 55, 60, 70 or 80 mg/mL. In yet another embodiment, the stabilizer comprises sucrose at about 20-60 mg/mL, e.g., sucrose at 20, 30, 40, 50 or 60 mg/mL. In one preferred embodiment, the stabilizer further comprises arginine, e.g., at about 25-200 mM, e.g., 50-150 mM, or 50-120 mM, preferably at about 60-100 mM, e.g., arginine at about 60, 65, 70, 75, 80, 85 or 90 mM. Preferably, arginine as the stabilizer is provided by arginine hydrochloride.

In one preferred embodiment, the stabilizer comprises a combination of sorbitol and arginine; or a combination of sucrose and arginine. For example, a combination of sorbitol at about 20-40 mg/mL and arginine at about 50-100 mM, or a combination of sucrose at about 30-60 mg/mL and arginine at about 50-100 mM may be used.

In preferred embodiments, the liquid antibody formulation disclosed herein comprises sorbitol at about 30-60 mg/mL (e.g., about 50 mg/mL), or sorbitol at about 20-30 mg/mL (e.g., about 25 mg/mL) and arginine at 80-90 mM (about 85 mM).

In one embodiment, the surfactant in the liquid antibody formulation disclosed herein has a concentration of about 0.1-1 mg/mL. In one embodiment, the surfactant in the liquid antibody formulation disclosed herein has a concentration of about 0.2-0.8 mg/mL, e.g., about 0.2, 0.3, 0.4, 0.5, 0.6, 0.7 or 0.8 mg/mL.

In one embodiment, the surfactant is a nonionic surfactant. In one embodiment, the surfactant is selected from polysorbate surfactants. In one specific embodiment, the surfactant in the liquid antibody formulation disclosed herein is polysorbate 80.

In one embodiment, the liquid formulation has a pH of about 5.0-6.0. In some embodiments, the liquid formulation has a pH of any of about 5.0-6.0, e.g., about 5.0, 5.2, 5.4, 5.6, 5.8 or 6.0. Preferably, the formulation has a pH of 5.2 ± 0.2 or 5.5 ± 0.2, preferably a pH of 5.2.

In one embodiment, the liquid antibody formulation disclosed herein comprises:
(i) an anti-TIGIT antibody at about 10-100 mg/mL, for example, 10-80 mg/mL, e.g., antibody at 10, 20, 30, 40, 50, 60, 70 or 80 mg/mL;
(ii) a histidine buffer at about 5-50 mM, for example, 5-30 mM, e.g., 5, 10, 15, 20, 25 or 30 mM;
(iii) sorbitol, sucrose, or any combination thereof at about 50-300 mM, e.g., 50, 80, 100, 120, 140, 160, 180, 200, 250 or 300 mM;
(iv) polysorbate 80 at about 0.1-1 mg/mL, e.g., 0.1, 0.2, 0.3, 0.4, 0.5, 0.6, 0.7, 0.8, 0.9 or 1.0 mg/mL; and
(v) optionally, arginine at about 50-120 mM,
wherein the liquid formulation has a pH of about 5.0-5.5, e.g., about 5.2.

For example, the liquid antibody formulation may comprises:
(i) an anti-TIGIT antibody at about 10-60 mg/mL, e.g., 20, 30, 40, 50, 55 or 60 mg/mL;
(ii) a histidine buffer at about 10 mM;
(iii) sorbitol or sucrose at about 10-50 mg/mL, e.g., sorbitol at 10, 15, 20, 25, 30, 35, 40, 45 or 50 mg/mL, or e.g., sucrose at 10, 15, 20, 25, 30, 35, 40, 45 or 50 mg/mL;
(iv) polysorbate 80 at about 0.2-0.8 mg/mL, for example, 0.2, 0.3, 0.4, 0.5, 0.6, 0.7 or 0.8 mg/mL, e.g., 0.3-0.6 mg/mL; and
(v) arginine at about 60-100 mM, e.g., 60, 65, 70, 75, 80, 85, 90, 95 or 100 mM, particularly about 85 mM,
wherein the liquid formulation has a pH of about 5.0-5.5, e.g., about 5.2.

In one preferred embodiment, the liquid antibody formulation comprises:
(i) an anti-TIGIT antibody at about 50 mg/mL, histidine at about 0.21 mg/mL, histidine hydrochloride at about 1.81 mg/mL, sorbitol at about 25.00 mg/mL, arginine hydrochloride at about 17.91 mg/mL and polysorbate 80 at about 0.50 mg/mL, about pH 5.2;
(ii) an anti-TIGIT antibody at about 50 mg/mL, histidine at about 0.21 mg/mL, histidine hydrochloride at about 1.81 mg/mL, sorbitol at about 25.00 mg/mL, arginine hydrochloride at about 17.91 mg/mL and polysorbate 80 at about 0.20 mg/mL, about pH 5.2; or
(iii) histidine at about 0.21 mg/mL, histidine hydrochloride at about 1.81 mg/mL, sucrose at about 40.00 mg/mL, arginine hydrochloride at about 17.91 mg/mL and polysorbate 80 at about 0.20 mg/mL, pH 5.2.

The liquid formulation disclosed herein can be stably stored for a long period of time, e.g., at least 24 months or longer. In one embodiment, the liquid formulation disclosed herein can be stable after storage at about -80 °C to about 45 °C, e.g., -80 °C, about -30 °C, about -20 °C, about 0 °C, about 5 °C, about 25 °C, about 35 °C, about 38 °C, about 40 °C, about 42 °C or about 45 °C, for at least 10 days, at least 20 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months, or longer.

In one embodiment, the liquid formulation disclosed herein can be stably stored for at least 24 months. In another embodiment, the liquid formulation disclosed herein is stable at a temperature of at least 40 °C. In yet another embodiment, the liquid formulation disclosed herein remains stable at about 2-8 °C for at least 3 months, preferably at least 12 months, and more preferably at least 24 months. In one embodiment, the liquid formulation disclosed herein remains stable at room temperature or, e.g., about 25 °C, for at least 2 months, preferably at least 3 months, and more preferably at least 6 months. In yet another embodiment, the liquid formulation disclosed herein remains stable at about 40 °C for at least 2 weeks, preferably at least 1 month.

In one embodiment, the stability of the formulation can be indicated by detecting changes in the appearance, visible particles, protein content, turbidity, purity and/or charge variants of the formulation. In one embodiment, the stability of the liquid formulation disclosed herein can be detected in a forced high temperature stress test, e.g., after storage at 40 ± 2 °C for at least 1 week, 2 weeks or preferably 1 month, or in an accelerated test, e.g., after storage at 25 ± 2 °C for at least 1 month or 2 months, or in a long-term test, e.g., after storage at 5 ± 3 °C for at least 2 months or 3 months, or in a shaking test (e.g. shaking at room temperature in the dark at 650 r/min for 5 days), and/or in a freezing-thawing test (e.g. 6 freezing-thawing repeats at -30 °C/room temperature). In one embodiment, the stability of the liquid formulation disclosed herein is tested relative to an initial value, for example, an initial value on day 0 of storage, or an initial value prior to a shaking or freezing-thawing test.

In one embodiment, the stability of the liquid formulation disclosed herein is visually inspected after storage, after a shaking test or after a freezing-thawing test, wherein the liquid formulation disclosed herein remains a clear to slightly opalescent, colorless to pale yellow liquid free of particles in appearance. In one embodiment, no visible particles exist in the formulation upon visual inspection under a clarity detector. In one embodiment, the stability of the liquid formulation disclosed herein is tested after storage, after a shaking test or after a freezing-thawing test by determining the change in protein content, wherein the change rate in protein content is not more than 20%, preferably not more than 10%, e.g., 7%-8%, and more preferably not more than 5%, 2% or 1%, relative to an initial value, as measured, for example, by ultraviolet spectrophotometry (UV). In one embodiment, the stability of the liquid formulation disclosed herein is tested after storage, after a shaking test or after a freezing-thawing test by determining the change in turbidity of the liquid formulation disclosed herein, wherein the change is not more than 0.06, preferably not more than 0.05, and more preferably not more than 0.04 or not more than 0.02, relative to an initial value, as measured, for example, by the OD_{350 nm} method. In one embodiment, the stability of the liquid formulation disclosed herein is tested after storage, after a shaking test or after a freezing-thawing test by determining the change in purity of the liquid formulation disclosed herein, wherein the change in monomer purity (or change in main peak) is not more than 10%, e.g., not more than 5%, 4% or 3%, e.g., not more than 2%, preferably no more than 1%, relative to an initial value, as measured by size exclusion chromatography-high performance liquid chromatography (SEC-HPLC). In one embodiment, the stability of the liquid formulation disclosed herein is tested after storage, after a shaking test or after a freezing-thawing test by determining the change in purity of the formulation disclosed herein, wherein the change in monomer purity (or change in main peak) is reduced by no more than 10%, e.g., no more than 5%, 4%, 3%, 2% or 1%, relative to an initial value, as measured by non-reduced capillary electrophoresis-sodium dodecyl sulfate (CE-SDS). In one embodiment, the stability of the liquid formulation disclosed herein is tested after storage, after a shaking test or after a freezing-thawing test by cation exchange high performance liquid chromatography (CEX-HPLC), wherein the sum of change in charge variants (principal component, acidic component and basic component) of the antibody is not more than 50%, e.g., not more than 40%, 30%, 20%, 10% or 5%, and/or the change in principal component is not more than 20%, 15%, 10%, 8% or 5%, relative to an initial value. In one embodiment, the stability of the liquid formulation disclosed herein is tested after storage, after a shaking test or after a freezing-thawing test by direct ELISA, wherein the relative binding activity of the antibody is 70%-130%, e.g. 70%, 80%, 90%, 93%, 95%, 98%, 100%, 103%, 105%, 108%, 110%, 115%, 120%, 125% or 130%, preferably 90%-110%, relative to an initial value.

In one embodiment, the liquid formulation disclosed herein is stable after storage, e.g., at 25 °C for at least 2 months or at 40 ± 2 °C for 1 month, and preferably, has one or more of the following characteristics relative to an initial value on day 0 of storage:
(i) a main peak change less than 1%, and/or a purity greater than 96%, preferably greater than 97% or 98% as measured by SEC-HPLC;
(ii) a main peak change less than 2%, and/or a purity greater than 96%, preferably greater than 97% or 98% as measured by non-reduced CE-SDS;
(iii) a sum of change in components (principal component, acidic component and basic component) not more than 40% and/or a change in the principal component not more than 20% of the anti-TIGIT antibody in the formulation as measured by CEX-HPLC,
   for example, a sum of change not more than about 40% (e.g., not more than 35%, 30%, 25%, 20%, 15% or 10%) or a change in principal component not more than 20% (e.g., not more than 15%, 12%, 10% or 8%) after storage at 40 ± 2 °C for 1 month, or
   for example, a sum of change not more than about 20% (e.g., not more than 15%, 14%, 13% or 12%) or a change in principal component not more than about 15% (e.g., not more than 10%, 8%, 7%, 6% or 5%) after storage at 25 °C for 2 months; or
(iv) a relative binding activity of the anti-TIGIT antibody in the formulation of 70%-130%, for example, 90%, 93%, 95%, 98%, 100%, 103%, 105%, 108%, 110%, 115% or 120%, e.g., 90%-110%, as measured by ELISA.

In one preferred embodiment, the liquid formulation disclosed herein is stable at shaking and/or repeated freezing-thawing.

Preferably, the formulation is stable at shaking or repeated freezing-thawing, e.g. after shaking at room temperature in the dark at 650 r/min for 5 days or after 6 freezing-thawing repeats at -30 °C/room temperature, and has one or more of the following characteristics:
(i) a main peak change less than 1%, and/or a purity greater than 96%, preferably greater than 97%, 98% or 99% as measured by SEC-HPLC;
(ii) a main peak change less than 1%, and/or a purity greater than 96%, preferably greater than 97% or 98% as measured by non-reduced CE-SDS;
(iii) a sum of change in components (principal component, acidic component and basic component) not more than 2% of the anti-TIGIT antibody in the formulation as measured by CEX-HPLC; or
(iv) a relative binding activity of the anti-TIGIT antibody in the formulation of 70%-130%, e.g., 90%-110%, as measured by ELISA.

In one aspect, the liquid formulation disclosed herein is a pharmaceutical formulation, preferably an injection, and more preferably a subcutaneous injection or an intravenous injection. In one embodiment, the liquid formulation is an intravenous infusion.

In another aspect, the present invention provides a solid antibody formulation obtained by solidifying the liquid antibody formulation disclosed herein. The solidification treatment is implemented by, e.g., crystallization, spray drying, or freeze drying. In one preferred embodiment, the solid antibody formulation is, e.g., in the form of lyophilized powder for injection. The solid antibody formulation can be reconstituted in a suitable vehicle prior to use to give a reconstituted formulation of the present invention. The reconstituted formulation is also a liquid antibody formulation disclosed herein. In one embodiment, the suitable vehicle is selected from water for injection, organic solvents for injection (including but not limited to, oil for injection, ethanol, propylene glycol, and the like), and combinations thereof.

In one aspect, the present invention provides a delivery device comprising the liquid antibody formulation or the solid antibody formulation disclosed herein. In one embodiment, the delivery device disclosed herein is provided in the form of a pre-filled syringe comprising the liquid antibody formulation or the solid antibody formulation disclosed herein, e.g., for use in intravenous, subcutaneous, intradermal or intramuscular injection, or intravenous infusion.

In another aspect, the present invention provides a method for delivering the anti-TIGIT antibody to a subject, e.g., a mammal, comprising administering the liquid antibody formulation or the solid antibody formulation disclosed herein to the subject, the delivery being implemented, e.g., using a delivery device in the form of a pre-filled syringe.

In another aspect, the present invention provides use of the liquid antibody formulation or the solid antibody formulation disclosed herein in preparing a delivery device, a pre-filled syringe or a medicament that blocks binding of TIGIT to CD155 to reduce or eliminate the immunosuppressive effect of TIGIT in a subject, or in preparing a delivery device (e.g., a pre-filled syringe) or a medicament for treating or preventing a tumor or a pathogen infection in a subject, wherein, for example, the tumor is a cancer, including but not limited to a cancer in gastrointestinal tract, e.g., colon cancer.

The present invention further provides a method for blocking binding of TIGIT to CD155 to reduce or eliminate the immunosuppressive effect of TIGIT in a subject by administering to the subject the liquid antibody formulation or the solid antibody formulation disclosed herein or a delivery device (e.g., a pre-filled syringe) or a medicament comprising the liquid antibody formulation or the solid antibody formulation.

The present invention further provides a method for treating a disease, e.g., the tumor or the pathogen infection described above, in a subject by administering to the subject the liquid antibody formulation or the solid antibody formulation disclosed herein or a delivery device (e.g., a pre-filled syringe) or a medicament comprising the liquid antibody formulation or the solid antibody formulation.

Other embodiments of the present invention will become apparent by reference to the detailed description hereinafter.

### BRIEF DESCRIPTION OF THE DRAWINGS

The preferred embodiments of the present invention described in detail below will be better understood when read in conjunction with the following drawings. For the purpose of illustrating the present invention, currently preferred embodiments are shown in the drawings. However, it should be understood that the present invention is not limited to accurate arrangement and means of the embodiments shown in the drawings.
FIG. 1 illustrates the trend of change in antibody charge variant-principal component at different pH as measured by CEX-HPLC in the pH screening experiment in Example 1.
FIG. 2 illustrates the trend of change in antibody charge variant-acidic component at different pH as measured by CEX-HPLC in the pH screening experiment in Example 1.
FIG. 3 illustrates the trend of change in antibody charge variants as measured by CEX-HPLC in the stability confirmation experiment at 40 °C in Example 2.
FIG. 4 illustrates the trend of change in antibody charge variants as measured by CEX-HPLC in the stability confirmation experiment at 25 °C in Example 2.

### DETAILED DESCRIPTION

Before the present invention is described in detail, it should be understood that the present invention is not limited to the particular methods or experimental conditions described herein since the methods and conditions may vary. Further, the terms used herein are for the purpose of describing particular embodiments only and are not intended to be limiting.

### Definitions

Unless otherwise defined, all technical and scientific terms used herein have the same meaning as those commonly understood by those of ordinary skill in the art. For the purposes of the present invention, the following terms are defined below.

The term "about" used in combination with a numerical value is intended to encompass the numerical values in a range from a lower limit less than the specified numerical value by 5% to an upper limit greater than the specified numerical value by 5%.

The term "and/or", when used to connect two or more options, should be understood to refer to any one of the options or any two or more of the options.

As used herein, the term "comprise" or "comprising" is intended to include the described elements, integers or steps, but not to exclude any other elements, integers or steps. As used herein, the term "comprise" or "comprising", unless indicated otherwise, also encompasses the situation where the entirety consists of the described elements, integers or steps. For example, when referring to an antibody variable region "comprising" a particular sequence, it is also intended to encompass an antibody variable region consisting of the particular sequence.

As used herein, TIGIT refers to a "T-cell immune receptor with Ig and ITIM domains". The term also includes variants, isotypes, homologs and species homologs of TIGIT. TIGIT is also known as VSIG9, VSTM3 and WUCAM. Amino acid and nucleic acid sequences of human and murine versions of GITR can be found in GenBank Accession Nos. NP_776160 (human amino acid sequence) and NP_001139797 (murine amino acid sequence). TIGIT proteins may also include fragments of TIGIT, such as fragments comprising extracellular domains, e.g., fragments that retain the ability of binding to any of the antibodies disclosed herein.

As used herein, the term "antibody" is used in the broadest sense, and refers to a protein comprising an antigen-binding site and encompasses natural and artificial antibodies with various structures, including but not limited to intact antibodies and antigen-binding fragments of antibodies.

The terms "whole antibody", "full-length antibody", "complete antibody" and "intact antibody" are used interchangeably herein to refer to a glycoprotein comprising at least two heavy chains (H) and two light chains (L) interconnected by disulfide bonds. Each heavy chain consists of a heavy chain variable region (abbreviated herein as VH) and a heavy chain constant region. Each heavy chain constant region consists of 3 domains CHI, CH2 and CH3. Each light chain consists of a light chain variable region (abbreviated herein as VL) and a light chain constant region. Each light chain constant region consists of one domain CL. The VH region and the VL region can be further divided into hypervariable regions (complementarity determining regions, or CDRs), with relatively conservative regions (framework regions, or FRs) inserted therebetween. Each VH or VL consists of three CDRs and four FRs, arranged from amino-terminus to carboxyl-terminus in the following order: FR1, CDR1, FR2, CDR2, FR3, CDR3 and FR4. The constant regions are not directly involved in binding of antibodies to antigens, but exhibit a variety of effector functions.

The terms "human antibody" and "fully humanized antibody" are used interchangeably herein and refer to an antibody comprising variable regions in which both framework regions and CDR regions are derived from human germline immunoglobulin sequences. Furthermore, if the antibody comprises constant regions, the constant regions are also derived from human germline immunoglobulin sequences. The human antibody disclosed herein may comprises amino acid sequences that are not encoded by human germline immunoglobulin sequences (e.g., having mutations introduced by *in vitro* random or site-specific mutagenesis or *in vivo* somatic mutation), for example, in CDRs, particularly in CDR3. However, as used herein, the term "human antibody" does not include antibodies in which CDR sequences derived from the germline of other mammalian species (e.g., mice) are grafted into human framework sequences.

The term "antibody formulation" refers to a preparation in a form that allows the biological activity of an antibody as an active ingredient to be exerted effectively, and does not contain other components having unacceptable toxicity to a subject to which the formulation is to be administered. Such antibody formulations are generally sterile. Generally, the antibody formulation comprises a pharmaceutically acceptable excipient. A "pharmaceutically acceptable" excipient is an agent that can be reasonably administered to a mammal subject so that an effective dose of the active ingredient used in the formulation can be delivered to the subject. The concentration of the excipient is adapted to the mode of administration and may, for example, be acceptable for injection.

The term "anti-TIGIT antibody formulation", herein also referred to as the "antibody formulation disclosed herein", refers to a preparation comprising an anti-TIGIT antibody as an active ingredient and a pharmaceutically acceptable excipient. The anti-TIGIT antibody, as the active ingredient, is suitable for therapeutic or prophylactic administration to a human or non-human animal after the anti-TIGIT antibody is combined with the pharmaceutically acceptable excipient. The antibody formulation disclosed herein can be prepared, for example, as an aqueous liquid formulation, e.g., in a ready-to-use pre-filled syringe, or as a lyophilized formulation to be reconstituted (i.e., redissolved) by dissolution and/or suspension in a physiologically acceptable solution immediately prior to use. In some embodiments, the anti-TIGIT antibody formulation is in the form of a liquid formulation.

A "stable" antibody formulation refers to a formulation where the antibody retains an acceptable degree of physical and/or chemical stability after storage in specific conditions, after shaking and/or after repeated freezing-thawing. Although the antibody in the antibody formulation may not maintain 100% of its chemical structure after storage for a specific period of time, shaking or repeated freezing-thawing, the antibody formulation is considered "stable" when about 90%, about 95%, about 96%, about 97%, about 98%, or about 99% of the antibody structure or function is generally maintained after storage for a specific period of time. In some specific embodiments, the antibody aggregation or degradation or chemical modification is barely detected in the anti-TIGIT antibody formulation disclosed herein during manufacture, formulation, transportation and long-term storage, resulting in little or even no loss of biological activity of the anti-TIGIT antibody and high stability. In some embodiments, the anti-TIGIT antibody formulation disclosed herein substantially retains its physical and chemical stability after storage, shaking and/or repeated freezing-thawing. Preferably, the liquid formulation disclosed herein can be stable at room temperature or at 40 °C for at least 2 weeks, and/or at 25 °C for at least 2 months, and/or at 2-8 °C for at least 24 months. Preferably, the liquid formulation disclosed herein can be stable after shaking at room temperature in the dark at 650 r/min for 5 days and/or after 1-6 freezing-thawing repeats at -30 °C/room temperature.

A variety of analytical techniques are known in the art for determining the stability of proteins, see, e.g., Peptide and Protein Drug Delivery, 247-301, Vincent Lee Ed., Marcel Dekker, Inc., New York, N.Y., Pubs (1991) and Jones, A. Adv. Drug Delivery Rev. 10: 29-90 (1993). Stability can be determined at a selected temperature and for a selected storage time. For example, the storage time can be selected based on the expected shelf life of the formulation. Alternatively, an accelerated stability test can be adopted. In some embodiments, the stability test is performed by conducting various stress tests on the antibody formulation. These tests can represent extreme conditions that a formulated antibody formulation may encounter during manufacture, storage or transportation, and can also represent conditions that may accelerate the instability of the antibody in the antibody formulation during a non-manufacture, -storage or -transportation process. For example, the formulated anti-TIGIT antibody formulation can be filled into a glass vial to test the stability of the antibody under high temperature stress. For another example, the stability of the antibody can be tested by filling the formulated anti-TIGIT antibody formulation into a glass vial and shaking at room temperature in the dark at 650 r/min for 5 days. For another example, the stability of the antibody can be tested by filling the formulated anti-TIGIT antibody formulation into a glass vial and subjecting to 1-6 freezing-thawing repeats at -30 °C/room temperature. In one embodiment, a 1-day cryopreservation below -30 °C and a following thawing at room temperature constitute a freezing-thawing cycle.

The antibody can be considered to "maintain its physical stability" in the formulation if the formulation does not exhibit aggregation, precipitation, turbidity and/or denaturation, or exhibits very little aggregation, precipitation, turbidity, and/or denaturation after a period of storage, after a period of shaking, or after repeated freeze-thawing. Safety issues arise as the aggregation of antibodies in the formulation can potentially lead to an increased immune response in a patient. Accordingly, there is a need to minimize or prevent the aggregation of antibodies in the formulation. Light scattering methods can be used to determine visible aggregates in the formulation. SEC-HPLC can be used to determine soluble aggregates in the formulation. In addition, the stability of the formulation can be indicated by visually inspecting the appearance, color and/or clarity of the formulation, by detecting the turbidity of the formulation by the OD_{350 nm} method, or by determining the purity of the formulation by non-reduced CE-SDS. In one embodiment, the stability of the formulation is measured by determining the percentage of antibody monomer in the formulation after storage at a particular temperature for a particular period of time, after shaking or after repeated freezing-thawing, wherein a higher percentage of antibody monomer in the formulation indicates a higher stability of the formulation.

An "acceptable degree" of physical stability can represent that at least about 90% of anti-TIGIT antibody monomer is detected in the formulation after storage at a specific temperature for a specific period of time, after shaking or after repeated freezing-thawing. In some embodiments, an acceptable degree of physical stability represents at least about 90%, 91%, 92%, 93%, 94%, 95%, 96%, 97%, 98% or 99% of anti-TIGIT antibody monomer after storage at a specific temperature for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer. When the physical stability is assessed, the specific temperature at which the pharmaceutical formulation is stored can be any temperature from about -80 °C to about 45 °C, e.g., about -80 °C, about -30 °C, about -20 °C, about 0 °C, about 4-8 °C, about 5 °C, about 25 °C, about 35 °C, about 37 °C, about 40 °C, about 42 °C, or about 45 °C. For example, a pharmaceutical formulation is considered stable if at least about 90%, 91%, 92%, 93%, 94%, 95%, 96%, 97%, 98% or 99% of anti-TIGIT antibody monomer is detected after storage at about 40 ± 2 °C for 1 month or 4 weeks. A pharmaceutical formulation is considered stable if at least about 90%, 91%, 92%, 93%, 94%, 95%, 96%, 97%, 98% or 99% of anti-TIGIT antibody monomer is detected after storage at about 25 °C for 2 months. A pharmaceutical formulation is considered stable if at least about 90%, 91%, 92%, 93%, 94%, 95%, 96%, 97%, 98% or 99% of anti-TIGIT antibody monomer is detected after storage at about 5 °C for 9 months.

The antibody can be considered to "maintain its chemical stability" in the formulation if the antibody in the formulation does not exhibit significant chemical changes after storage for a period of time, after shaking or after repeated freezing-thawing. Most of the chemical instability results from the formation of covalently modified forms of the antibody (e.g., charge variants of the antibody). Basic variants can be formed, for example, by aspartic acid isomerization, and N- and C-terminal modifications; acidic variants can be produced by deamidation, sialylation and saccharification. Chemical stability can be assessed by detecting and/or quantifying chemically altered forms of the antibody. For example, charge variants of the antibody in the formulation can be detected by cation exchange chromatography (CEX) or imaged capillary isoelectric focusing (iCIEF). In one embodiment, the stability of the formulation is measured by determining the percentage change in charge variants of the antibody in the formulation after storage at a specific temperature for a specific period of time, after shaking or after repeated freezing-thawing, wherein a smaller change indicates a higher stability of the formulation.

An "acceptable degree" of chemical stability can represent a percentage change in charge variants (e.g., principal component, acidic component or basic component) in the formulation not more than 40%, e.g., not more than 30% or 20%, or a sum of percentage change in charge variants (principal component, acidic component and basic component) not more than 60%, e.g., not more than 50% or 30%, after storage at a specific temperature for a specific period of time, after shaking or after repeated freezing-thawing. In some embodiments, an acceptable degree of chemical stability can represent a percentage change in charge variant-principal component not more than about 50%, 40%, 30%, 20% or 15% or a sum of percentage change in charge variants not more than about 60%, 50% or 30%, after storage at a specific temperature for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer. When the chemical stability is assessed, the temperature at which the pharmaceutical formulation is stored can be any temperature from about -80 °C to about 45 °C, e.g., about -80 °C, about -30 °C, about -20 °C, about 0 °C, about 4-8 °C, about 5 °C, about 25 °C, or about 45 °C. For example, the pharmaceutical formulation can be considered stable if the percentage change in charge variant-principal component is less than about 25%, 24%, 23%, 22%, 21%, 20%, 19%, 18%, 17%, 16%, 15%, 14%, 13%, 12%, 10%, 9%, 8%, 7%, 6%, 5%, 4%, 3%, 2%, 1%, 0.5% or 0.1% after storage at 5 °C for 24 months. The pharmaceutical formulation can also be considered stable if the percentage change in charge variant-principal component is less than about 20%, 19%, 18%, 17%, 16%, 15%, 14%, 13%, 12%, 10%, 9%, 8%, 7%, 6%, 5%, 4%, 3%, 2%, 1%, 0.5% or 0.1% after storage at 25 °C for 2 months. The pharmaceutical formulation can also be considered stable if the percentage change in charge variant-principal component is less than about 50%, 40%, 30%, 20%, 16%, 15%, 14%, 13%, 12%, 10%, 5% or 4% after storage at 40 °C for 1 month.

The term "lyophilized formulation" refers to a composition obtained or obtainable by a freeze-drying process of a liquid formulation. Preferably, it is a solid composition having a water content less than 5%, preferably less than 3%.

The term "reconstituted formulation" refers to a liquid formulation obtained by dissolving and/or suspending a solid formulation (e.g., a lyophilized formulation) in a physiologically acceptable solution.

The term "room temperature" as used herein refers to a temperature of 15-30 °C, preferably 20-27 °C, and more preferably 25 °C.

"Stress conditions" refer to environments that are chemically and/or physically unfavorable to antibody proteins and may result in unacceptable destabilization of the antibody, e.g., high temperature, shaking and freezing-thawing. "High temperature stress" refers to storing the antibody formulation at room temperature or higher (e.g., 40 ± 2 °C) for a period of time. The stability of the antibody formulation can be tested by a high-temperature stress accelerated test.

As used herein, the term "parenteral administration" refers to administrations other than intraintestinal and topical administrations, typically by injection or infusion, including but not limited to, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. In some embodiments, the stable anti-TIGIT antibody formulation disclosed herein is administered parenterally to a subject. In one embodiment, the anti-TIGIT antibody formulation is administered by subcutaneous, intradermal, intramuscular, or intravenous injection to a subject.

### I. Antibody Formulation

The present invention provides a stable liquid antibody formulation comprising (i) an anti-TIGIT antibody, (ii) a buffer, (iii) a stabilizer, and (iv) a surfactant, wherein the antibody formulation is at about pH 5.0-6.0. In one preferred embodiment, the liquid antibody formulation disclosed herein is in the form of an injection.

### (i) Anti-TIGIT antibody

An "anti-TIGIT antibody" refers to an antibody that is capable of binding to a TIGIT molecule with sufficient affinity such that the antibody can be used as a therapeutic agent and/or prophylactic agent targeting the TIGIT molecule.

In some embodiments, the anti-TIGIT antibody in the antibody formulation disclosed herein can specifically bind to human TIGIT with a high affinity, e.g., with K_{D} of 10⁻⁷ M or less, preferably 10-20 × 10⁻¹⁰ M as measured by biological optical interferometry, and thus mediates the efficient blocking of TIGIT binding to its ligand CD155 and reduces or eliminates the inhibitory signaling caused by the binding of TIGIT to its ligand. In some embodiments, the anti-TIGIT antibody in the antibody formulation disclosed herein inhibits the growth of tumors containing infiltrating lymphocytes that express human TIGIT (e.g., tumors in gastrointestinal tract, preferably colorectal cancer), and preferably when used in combination with an anti-PDl antibody, achieves a significantly better anti-tumor effect than use alone of either antibody.

In some embodiments, the anti-TIGIT antibody in the antibody formulation disclosed herein comprises: a heavy chain variable region (VH) of SEQ ID NO: 7 or a VH having at least 90% identity thereto; and a light chain variable region (VL) of SEQ ID NO: 8 or a VL having at least 90% identity thereto. A "variable region" or "variable domain" is a domain in the heavy chain or light chain of an antibody that participates in binding of the antibody to the antigen thereof. Generally, a heavy chain variable region (VH) and a light chain variable region (VL) can be further divided into hypervariable regions (HVRs, also known as complementarity determining regions (CDRs)) with more conservative regions (i.e., framework regions (FRs)) inserted therebetween. Each VH or VL consists of three CDRs and four FRs, arranged from amino-terminus to carboxyl-terminus in the following order: FR1, CDR1, FR2, CDR2, FR3, CDR3 and FR4.

In some embodiments, the anti-TIGIT antibody in the antibody formulation disclosed herein comprises the VH CDR1, VH CDR2 and VH CDR3 sequences of the heavy chain variable region of SEQ ID NO: 7, and the VL CDR1, VL CDR2 and VL CDR3 sequences of the light chain variable region of SEQ ID NO: 8. "Complementarity determining region", "CDR region" or "CDR" (used interchangeably herein with a "hypervariable region" (HVR)) is an amino acid region in the variable region of an antibody that is primarily responsible for binding to an epitope of an antigen. The CDRs of the heavy and light chains are generally referred to as CDR1, CDR2, and CDR3, and are numbered sequentially from the N-terminus. The CDRs located in the variable domain of an antibody heavy chain are referred to as VH CDR1, VH CDR2 and VH CDR3, while the CDRs located in the variable domain of an antibody light chain are referred to as VL CDR1, VL CDR2, and VL CDR3. Various schemes for determining the CDR sequence of a given VH or VL amino acid sequence are known in the art. For example, Kabat complementarity determining regions (CDRs) are determined based on sequence variability and are the most commonly used (Kabat et al., Sequences of Proteins of Immunological Interest, 5th Ed. Public Health Service, National Institutes of Health, Bethesda, Md. (1991)). Chothia scheme is based on the positions of structural loops (Chothia and Lesk, J. mol. biol. 196:901-917 (1987)). AbM HVRs are a compromise between Kabat HVRs and Chothia structural loops and are used by Oxford Molecular's AbM antibody modeling software. The "contact" HVRs are based on analysis of available complex crystal structures. HVRs can also be determined based on the same Kabat numbering position as the reference CDR sequences (e.g., exemplary CDRs disclosed herein). In one embodiment, the anti-TIGIT antibody disclosed herein comprises a VH CDR1 of SEQ ID NO: 1, a VH CDR2 of SEQ ID NO: 2 and a VH CDR3 of SEQ ID NO: 3; and a VL CDR1 of SEQ ID NO: 4, a VL CDR2 of SEQ ID NO: 5 and a VL CDR3 of SEQ ID NO: 6.

In some embodiments, the anti-TIGIT antibody in the antibody formulation disclosed herein can comprise a heavy chain variable region (VH) having at least 90%, 95%, 98%, or 99% or higher identity to SEQ ID NO: 7; and/or a light chain variable region (VL) having at least 90%, 95%, 98%, or 99% or higher identity to SEQ ID NO: 8. As used herein, the term "sequence identity" refers to the degree to which sequences are identical on a nucleotide-by-nucleotide or amino acid-by-amino acid basis in a comparison window. The "percent sequence identity" can be calculated by the following steps: comparing two optimally aligned sequences in a comparison window; determining a number of positions in which nucleic acid bases (e.g., A, T, C, G and I) or amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are the same in the two sequences to give the number of matched positions; dividing the number of matched positions by the total number of positions in the comparison window (i.e., the window size); and multiplying the result by 100 to give a percent sequence identity. Optimal alignment for determining the percent sequence identity can be achieved in a variety of ways known in the art, for example, using publicly available computer software such as BLAST, BLAST-2, ALIGN, or Megalign (DNASTAR) software. Those skilled in the art can determine suitable parameters for alignment of the sequences, including any algorithms necessary to achieve optimal alignment in a full-length sequence range or target sequence region being compared.

In some embodiments, the VH sequence of the antibody disclosed herein has no more than 10, preferably no more than 5, 4 or 3 different residues as compared to SEQ ID NO: 7, wherein preferably, the different residues are conservative amino acid substitutions. In some embodiments, the VL sequence of the antibody disclosed herein has no more than 10, preferably no more than 5, 4 or 3 different residues as compared to SEQ ID NO: 8, wherein preferably, the different residues are conservative amino acid substitutions. The "conservative substitution" refers to an amino acid alteration that results in the replacement of an amino acid with a chemically similar amino acid. Conservative substitution tables providing functionally similar amino acids are well known in the art. In any of the embodiments herein, in one preferred aspect, the conservatively substitution residue is from the conservative substitution Table A below, preferably the preferred substitution residues shown in Table A.

**Table A**

| Original residues | Exemplary replacement | Preferred conservative amino acid replacement |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Nle | Leu |
| Leu (L) | Nle; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Nle | Leu |

In some embodiments, the anti-TIGIT antibody in the antibody formulation disclosed herein is an antibody in the form of IgG. "Antibody in the form of IgG" refers to the heavy chain constant region of the antibody belonging to the IgG form. Heavy chain constant regions of all antibodies of the same IgG subtype are identical, and heavy chain constant regions of antibodies of different IgG subtypes are different. For example, an antibody in the form of IgG1 refers to the Ig domain of its heavy chain constant region being an Ig domain of IgG1.

In one preferred embodiment, the anti-TIGIT antibody in the antibody formulation disclosed herein is the anti-TIGIT monoclonal antibody ADI-30278 disclosed in PCT Application No. PCT/CN2019/097665 (International Application Date: Jul. 25, 2019), having a heavy chain of SEQ ID NO: 9 and a light chain of SEQ ID NO: 10. In one embodiment, the anti-TIGIT antibody is an isolated IgG4 antibody produced by recombinant expression in CHO cells. Preferably, the antibody in the liquid formulation disclosed herein exhibits significant anti-tumor activity. For example, in a mouse tumor model bearing mouse MC38 cells, administration of the antibody formulation disclosed herein may result in a significant tumor suppressive effect, particularly in combination with an anti-PD-1 antibody.

The amount of antibody or antigen-binding fragment thereof in the antibody formulation disclosed herein can vary with the specific desired characteristics of the formulation, the specific environment, and the specific purpose for which the formulation is used. In some embodiments, the antibody formulation is a liquid formulation, which may comprise about 1-150 mg/mL, preferably about 10-100 mg/mL, e.g., about 10, 15, 20, 25, 30, 35, 40, 50, 60, 70, 80, 90 or 100 mg/mL of the anti-TIGIT antibody.

### (ii) Buffer

Buffers are reagents that can control the pH of a solution within an acceptable range. In some embodiments, the buffer in the formulation disclosed herein can control a pH of the formulation disclosed herein at about 5.0-6.0, e.g., about 5.0-5.5. In some specific embodiments, the antibody formulation disclosed herein has a pH of about 5.0, 5.1, 5.2, 5.3, 5.4, 5.5, 5.6, 5.7 or 5.8. For example, the antibody formulation disclosed herein has a pH of 5.2 ± 0.2 or 5.5 ± 0.2, preferably a pH of 5.2.

In some embodiments, the formulation disclosed herein comprise a buffer system selected from: a histidine-histidine hydrochloride buffer system, a citric acid-sodium citrate buffer system, an acetic acid-sodium acetate buffer system and a phosphate buffer system, preferably, a histidine-histidine hydrochloride buffer system. In some embodiments, the buffer used in the formulation of the present invention is a histidine buffer, particularly a buffer system consisting of histidine and histidine hydrochloride. In some embodiments, the concentration of histidine in the histidine buffer of the present invention is about 5-50 mM, particularly about 5-30 mM, e.g., about 5, 10, 15, 20, 25, 30 mM. In one embodiment, the formulation disclosed herein comprises histidine at about 10 mM. In another embodiment, the histidine buffer used in the formulation disclosed herein consists of, for example, about 0.21 mg/mL of histidine and about 1.81 mg/mL of histidine hydrochloride.

### (iii) Stabilizer

Suitable stabilizers for use in the present invention can be selected from saccharides, polyols and amino acids and combinations thereof. Saccharides that may be used as a stabilizer include, but are not limited to, sucrose, trehalose, maltose, and a combination thereof. Polyols that may be used as a stabilizer include, but are not limited to, sorbitol, mannitol, and a combination thereof. Amino acids that may be used as a stabilizer include, but are not limited to, arginine, arginine hydrochloride, methionine, glycine, proline, and a combination thereof.

For example, in some embodiments, the stabilizer comprises one or more selected from:
- a polyol selected from sorbitol, mannitol and a combination thereof, about 10-100 mg/mL, preferably 20-40 mg/mL, e.g., 25 mg/mL; or 40-60 mg/mL, e.g., 50 mg/mL;
- a saccharide selected from sucrose, trehalose, maltose and a combination thereof, for example about 10-100 mg/mL, preferably 30-60 mg/mL, e.g., 40 mg/mL; and
- an amino acid selected from arginine hydrochloride, methionine, glycine, proline and a combination thereof, for example, 20-200 mM, e.g., about 50-110 mM, e.g. 70-100 mM, particularly about 80-90 mM.

In one embodiment, the liquid formulation disclosed herein comprises sucrose as the stabilizer. The amount of sucrose in the liquid formulation disclosed herein may be about 10-100 mg/mL, preferably 30-60 mg/mL, e.g. 40 mg/mL.

In one embodiment, the liquid formulation disclosed herein comprises sorbitol as the stabilizer. The amount of sorbitol in the liquid formulation disclosed herein may be about 10-100 mg/mL, preferably 20-40 mg/mL, e.g., 25 mg/mL; or 40-60 mg/mL, e.g., 50 mg/mL.

In one embodiment, the liquid formulation disclosed herein comprises arginine as the stabilizer. The amount of arginine in the liquid formulation disclosed herein may be about 50-110 mM, for example, 70-100 mM, particularly about 80-90 mM, e.g., arginine hydrochloride at about 17.91 mg/mL.

In one embodiment, the liquid formulation disclosed herein comprises sorbitol as the sole stabilizer. In this embodiment, the amount of sorbitol in the liquid formulation disclosed herein may be about 30-70 mg/mL, e.g., 40-60 mg/mL. For example, the amount of sorbitol may be about 30, 35, 40, 45, 50, 55, 60, 65 or 70 mg/mL, preferably about 50 mg/mL.

In one embodiment, the liquid formulation disclosed herein comprises a combination of sorbitol and arginine as the stabilizer. In the combination, the amount of sorbitol may be about 10-60 mg/mL, preferably 15-40 mg/mL, e.g., 20-35 mg/mL, e.g., about 20, 21, 22, 23, 24, 25, 26, 27, 28, 29 or 30 mg/mL. In the combination, the amount of arginine may be about 70-100 mM, particularly about 85 mM. Preferably, the liquid formulation disclosed herein comprises sorbitol at about 20-30 mg/mL and arginine hydrochloride at about 15-20 mg/mL. More preferably, the liquid formulation disclosed herein comprises sorbitol at about 25 mg/mL and arginine hydrochloride at about 17.91 mg/mL.

In one embodiment, the liquid formulation disclosed herein comprises a combination of sucrose and arginine as the stabilizer. In this combination, the amount of sucrose may be about 10-60 mg/mL, preferably 20-50 mg/mL, for example, 30-40 mg/mL, e.g. may be about 30, 32, 34, 36, 38, 40, 42, 44, 46, 48 or 50 mg/mL. In the combination, the amount of arginine may be about 70-100 mM, particularly about 85 mM. Preferably, the liquid formulation disclosed herein comprises sucrose at about 30-50 mg/mL and arginine hydrochloride at about 15-20 mg/mL. More preferably, the liquid formulation disclosed herein comprises sucrose at about 40 mg/mL and arginine hydrochloride at about 17.91 mg/mL.

### (iv) Surfactant

As used herein, the term "surfactant" refers to an organic substance with an amphiphilic structure; that is, the structure is composed of groups with opposite solubility tendencies, typically an oil-soluble hydrocarbon chain and a water-soluble ionic group.

In one embodiment, the surfactant in the liquid formulation disclosed herein is a non-ionic surfactant, e.g., alkyl poly(ethylene oxide). Specific non-ionic surfactants that can be contained in the formulation disclosed herein include, for example, polysorbates such as polysorbate 20, polysorbate 80, polysorbate 60 or polysorbate 40, poloxamer, and the like. In one preferred embodiment, the liquid formulation disclosed herein comprises polysorbate 80 as the surfactant.

In some embodiments, surfactants that can be used in the liquid formulation disclosed herein include, but are not limited to, polysorbate-based surfactants (e.g., polysorbate 80, polysorbate 20), poloxamer and polyethylene glycol.

The amount of the surfactant in the antibody formulation disclosed herein can vary with the specific desired characteristics of the formulation, the specific environment, and the specific purpose for which the formulation is used. In some preferred embodiments, the formulation may comprise a surfactant, particularly polysorbate 80, at about 0.01-5 mg/mL, preferably about 0.1-1 mg/mL, e.g., about 0.2, 0.3, 0.4, 0.5, 0.6, 0.7, 0.8, 0.9 or 1.0 mg/mL, preferably polysorbate 80 at about 0.5 mg/mL.

### (v) Other excipients

The liquid antibody formulation disclosed herein may or may not comprise other excipients. Such other excipients include, for example, antimicrobials, antistatic agents, antioxidants, chelating agents, gelatin, and the like. These and other known pharmaceutical excipients and/or additives suitable for use in the formulation disclosed herein are well known in the art, for example, as listed in *"*The Handbook of Pharmaceutical Excipients, 4th edition, edited by Rowe et al., American Pharmaceuticals Association (2003); and Remington: the Science and Practice of Pharmacy, 21st edition, edited by Gennaro, Lippincott Williams & Wilkins (2005)".

### II. Preparation of Formulation

The present invention provides a stable formulation comprising an anti-TIGIT antibody. The anti-TIGIT antibody used in the formulation disclosed herein can be prepared using techniques known in the art for the production of antibodies. For example, the antibody can be recombinantly prepared. In one preferred embodiment, the antibody disclosed herein is recombinantly prepared in 293 cells or CHO cells.

The use of antibodies as active ingredients in drugs is now very common. Techniques for purifying therapeutic antibodies to pharmaceutical grade are well known in the art. For example, Tugcu et al. (Maximizing productivity of chromatography steps for purification of monoclonal antibodies, Biotechnology and Bioengineering 99 (2008) 599-613) described a monoclonal antibody three-column purification method in which ion exchange chromatography (anionic IEX and/or cationic CEX chromatography) is used after a protein A capture step. Kelley et al. (Weak partitioning chromatography for anion exchange purification of monoclonal antibodies, Biotechnology and Bioengineering 101 (2008) 553-566) described a two-column purification method in which a weak partitioning anion exchange resin is used after protein A affinity chromatography.

Generally, monoclonal antibodies recombinantly produced can be purified using conventional purification methods to provide a drug substance with sufficient reproducibility and proper purity for the formulation of antibody formulations. For example, after the antibody is secreted from the recombinant expression cells into the culture medium, the supernatant of the expression system can be concentrated using a commercially available protein concentration filter, e.g., Amicon ultrafiltration device. Then the antibody can be purified by methods such as chromatography, dialysis and affinity purification. Protein A is suitable as an affinity ligand for the purification of IgG1, IgG2 and IgG4 antibodies. Other antibody purification methods, such as ion exchange chromatography, can also be used. After the antibody with sufficient purity is obtained, a formulation comprising the antibody can be prepared according to methods known in the art.

For example, the preparation can be performed by the following steps: (1) removing impurities such as cells from fermentation broth by centrifuging and clarifying after the fermentation to give a supernatant; (2) capturing an antibody using affinity chromatography (e.g., a protein A column with specific affinity for IgG1, IgG2 and IgG4 antibodies); (3) inactivating viruses; (4) purifying (usually CEX cation exchange chromatography can be adopted) to remove impurities in a protein; (5) filtering the viruses (to reduce the virus titer by, e.g., more than 4 log10); and (6) ultrafiltering/diafiltering (which can be used to allow the protein to be exchanged into a formulation buffer that is favorable for its stability and concentrated to a suitable concentration for injection). See, e.g., B. Minow, P. Rogge, K. Thompson, BioProcess International, Vol. 10, No. 6, 2012, pp. 48-57.

### III. Analytical Method of Formulation

Biologics stability studies typically include real-time stability studies in actual storage conditions (long-term stability studies), accelerated stability studies and forced condition studies. For the stability studies, the study conditions are explored and optimized according to the purpose and the characteristics of the product; stability study protocols, such as long-term, accelerated and/or forced condition studies and the like, should be established according to various influencing factors (such as temperature, repeated freezing-thawing, vibration and the like). Accelerated and forced condition studies are beneficial to understanding the stability of the product in short-term deviations from storage conditions and in extreme conditions, and provide supporting data for the determination of the shelf life and storage conditions.

During the storage, shaking or repeated freezing-thawing of antibody formulations, antibodies may undergo aggregation, degradation or chemical modification, resulting in antibody heterogeneity (including size heterogeneity and charge heterogeneity), aggregates and fragments, etc., which may affect the quality of the antibody formulations. Accordingly, it is necessary to monitor the stability of antibody formulations.

Various methods are known in the art for testing the stability of antibody formulations. For example, the purity of the antibody formulation can be analyzed and the aggregation level of the antibody can be evaluated by methods such as reduced CE-SDS, non-reduced CE-SDS and SEC-HPLC; charge variants in the antibody formulation can be analyzed by capillary isoelectric focusing electrophoresis (cIEF), imaged capillary isoelectric focusing (iCIEF), ion exchange chromatography (IEX), and the like. In addition, the stability of the formulation can be determined quickly by visually inspecting the appearance of the formulation. The change in turbidity of the formulation can also be detected by the OD_{350 nm} method, which gives information about the amount of soluble and insoluble aggregates. In addition, the change in protein content in the formulation can be detected by ultraviolet spectrophotometry (UV).

Non-reduced CE-SDS is a method for determining the purity of antibodies using a capillary as a separation channel. In CE-SDS, protein migration is driven by the surface charge caused by SDS binding, which is proportional to the molecular weight of the protein. Since all SDS-protein complexes have similar mass-to-charge ratios, electrophoretic separation based on the size or hydrodynamic radius of the molecules can be achieved in the molecular sieve gel matrix of the capillary. This method has been widely used to monitor the purity of denatured intact antibodies. Generally, in non-reduced CE-SDS, the test sample is mixed with an SDS sample buffer and iodoacetamide. Then the mixture can be incubated at 68-72 °C for about 10-15 min and cooled to room temperature before the supernatant is centrifuged for analysis. The protein migration is detected using an ultraviolet detector to give an electropherogram. The purity of the antibody formulation can be calculated as the percentage of the IgG main peak area to the sum of all peak areas. For further description of CE-SDS, see, e.g., Richard R. et al., Application of CE SDS gel in development of biopharmaceutical antibody-based products, Electrophoresis, 2008, 29, 3612-3620.

Size exclusion chromatography-high performance liquid chromatography (SEC-HPLC) is another important method for the standardization and quality control of antibodies. In this method, molecules are separated mainly based on the differences in their size or hydrodynamic radius. Antibodies can be separated in three main forms by SEC-HPLC: high-molecular-weight species (HMMS), main peak (mainly antibody monomer), and low-molecular-weight species (LMMS). The purity of the antibody can be calculated as the percentage of the main peak area to the sum of all peak areas on the chromatogram. The percentage of antibody monomer in the formulation can be measured by SEC-HPLC, which gives information about the content of soluble aggregates and splices. For further description of SEC-HPLC, see, e.g., J. Pharm. Scien., 83:1645-1650, (1994); Pharm. Res., 11:485 (1994); J. Pharm. Bio. Anal., 15:1928 (1997); J. Pharm. Bio. Anal., 14:1133-1140 (1986). In addition, see also, e.g., R. Yang et al., High resolution separation of recombinant monoclonal antibodies by size exclusion ultra-high performance liquid chromatography (SE-UHPLC), Journal of Pharmaceutical and Biomedical Analysis (2015), http://dx.doi.org/10.1016/j.jpba.2015.02.032; and Alexandre Goyon et al., Protocols for the analytical characterization of therapeutic monoclonal antibodies, I-Non-denaturing chromatographic techniques, Journal of Chromatography, http://dx.doi.org/10.1016/j.jchromb.2017.05.010.

The charge variants of the antibody in the antibody formulation can be determined by cation exchange high performance liquid chromatography (CEX-HPLC). In this method, peaks eluted from the CEX-HPLC column earlier than the retention time of the main peak (or principal component) are labeled as "acidic peaks" (or acidic component), while those eluted from the CEX-HPLC column later than the retention time of the main peak are labeled as "basic peaks" (or basic component).

Accelerated stability studies can be used to check the stability of products, which facilitates the screening of stable pharmaceutical formulations. For example, formulation samples can be placed at an elevated temperature, e.g., about 40 ± 2 °C or 25 ± 2 °C, for an accelerated stability study. In addition, shaking test or repeated freezing-thawing test can be conducted to test the stability properties of the product. For example, the shaking test is conducted at room temperature in the dark at 650 r/min for 1-5 days. For example, the repeated freezing-thawing experiment can be conducted by a cycle of a 1-day cryopreservation below -30 °C and a following thawing at room temperature, wherein 1-6 repeated freezing-thawing cycles may be performed. The test indexes for product stability may include appearance, visible particles, protein content, turbidity, purity (SEC-HPLC and non-reduced CE-SDS) and charge variants (iCIEF and CEX-HPLC). In addition, the efficacy or biological activity of the antibody can be detected. For example, the ability of the antibody in the formulation to bind to its antigenic molecule (TIGIT) can be tested. Various methods are known to those skilled in the art for quantifying the specific binding of an antibody to an antigen, such as immunoassay, e.g., ELISA.

### IV. Use of Formulation

The antibody formulation disclosed herein comprising the anti-TIGIT antibody of the present invention has an effect of reducing immunosuppression and can be used for treating or preventing tumors, pathogen infection, and the like.

In one embodiment, the formulation disclosed herein can be used to block binding of TIGIT to CD155 to reduce or eliminate the immunosuppressive effect of TIGIT in a subject. In another embodiment, the formulation disclosed herein may be used to treat or prevent tumor or pathogen infection in a subject. The tumor is, for example, a cancer in gastrointestinal tract, such as colon cancer.

In one embodiment, the formulation disclosed herein may be administered in combination with a second therapeutic agent, such as an anti-PD-1 antibody.

The present invention further provides use of the formulation disclosed herein in preparing a medicament for delivering an anti-TIGIT antibody to a mammal. The present invention further provides a method for treating or preventing one or more of the above diseases and disorders with the formulation disclosed herein. Preferably, the mammal is a human.

The antibody formulation disclosed herein can be administered to a subject or a patient in a variety of routes. For example, the administration can be performed by infusion or by using a syringe. Accordingly, in one aspect, the present invention provides a delivery device (e.g., a syringe) comprising the antibody formulation disclosed herein (e.g., a pre-filled syringe). The patient will receive an effective amount of the anti-TIGIT antibody as the primary active ingredient, i.e., an amount sufficient to treat, ameliorate or prevent the disease or disorder of interest.

The therapeutic effect can include a reduction in physiological symptoms. The optimal effective amount and concentration of the antibody for any specific subject will depend on a variety of factors including the age, weight, health status and/or sex of the patient, the nature and extent of the disease, the activity of the specific antibody, its clearance in the body, as well as any other possible treatments administered in combination with the antibody formulation. For a specific case, the effective amount delivered can be determined based on the judgment of a clinician.

The following examples are described to assist in understanding the present invention. The examples are not intended to be and should not be interpreted in any way as limiting the protection scope of the present invention.

### Abbreviations

| Abbreviation | Full version |
|---|---|
| CE-SDS | Capillary electrophoresis-sodium dodecyl sulfate |
| CEX-HPLC | Cation exchange high performance liquid chromatography |
| ELISA | Enzyme-linked immunosorbent assay |
| SEC-HPLC | Size exclusion chromatography-high performance liquid chromatography |

### Examples

The recombinant fully human anti-TIGIT monoclonal antibody ADI-30278, an antibody independently developed by the assignee Innovent Biologics (Suzhou) Co., Ltd., was disclosed in PCT Application No. PCT/CN2019/097665. The antibody can effectively block the binding of TIGIT to its ligand CD155, relieve the inhibitory effect of CD155 on a downstream IL2 signaling pathway, inhibit the growth of tumors when administered *in vivo,* and particularly have significant tumor inhibitory effect in combination with an anti-PD-1 antibody.

In order to develop a simple and easy-to-use injection formulation suitable for long-term stable storage of the fully human antibody, the influence of different pH values, different stabilizers and the content of surfactant on quality of the antibody was investigated by 40 °C forced and 25 °C accelerated stability tests, and finally, a formulation favorable for the stability of the antibody was selected. The materials and methods used throughout the study are as follows:

### Materials and methods

### 1.1. Materials

| Name | Grade | Manufacturer & brand | Catalog No. | Criteria |
|---|---|---|---|---|
| Histidine | Pharmaceutical grade | Merck, Germany | 1.04352.1000 | *Ch.P* (2015 Edition) |
| Histidine hydrochloride | Pharmaceutical grade | Merck, Germany | 1.04354.0500 | *Ch.P* (2015 Edition) |
| Sorbitol | Pharmaceutical grade | Roquette, French | H20110265 | *EP*, *BP, NF, USP, Ch. P* (2015 edition) |
| Sucrose | Pharmaceutical grade | Merck, Germany | 1.07653.9029 | *Ch.P* (2015 Edition), *USP* |
| Arginine hydrochloride | Pharmaceutical grade | Merck, Germany | 1.01544.1000 | *Ph Eur, BP, JP, USP, Ch.P* (2015 edition) |
| Polysorbate 80 | Pharmaceutical grade | Well, Nanjing | Jiangsu MPA Approval No. F15423203 | *Ch.P* (2015 Edition) |
| Hydrochloric acid | Pharmaceutical grade | Merck, Germany | 1.00314.2508 | *Ph Eur, BP, JP, NF, Ch.P* (2015 edition) |
| Capsule filter H4 | N/A | Sartorius, Germany | 5441307H4--OO--B | N/A |
| Platinum -cured silicone tubing | N/A | Nalgene, USA | 8600-0080 | N/A |
| 6R vial | N/A | Schott, Suzhou | 1142196 | N/A |
| 20 mm rubber stopper | N/A | West, Singapore | 7002-2354 | N/A |
| 20 mm aluminum -plastic cap | N/A | West, Singapore | 5420-1035 | N/A |

| | | | | |
|---|---|---|---|---|
| Note: N/A denotes not applicable. | | | | |

### 1.2. Instruments and equipment

| Name | Manufacturer & brand | Model No. | No. |
|---|---|---|---|
| Name | Manufacturer & brand | Model No. | No. |
| Electronic balance | Sartorius, Germany | BSA3202S | PD-A1-186 |
| Electronic balance | Mettler, Switzerland | XPE3003S | PD-A1-247 |
| Constant climate chamber | BINDER, Germany | KBF P 720 | PD-A1-070 |
| Biochemical incubator | Jinghong, Shanghai | SHP-150 | PD-A1-200 |
| Medical refrigerator | Haier, Qingdao | HYC-360 | PD-A1-166 |
| Medical refrigerator | Haier, Qingdao | HYC-360 | PD-A1-165 |
| Ultra-low temperature refrigerator | Thermo, USA | 907 | PD-A1-175 |
| Clarity detector | Tianda Tianfa, Tianjin | YB-2 | PD-A1-033 |
| Ultraviolet-visible spectrophotometer | Shimadzu, Japan | UV-1800 | AS-A1-037 |
| pH meter | Mettler, Switzerland | FE20 | PD-A1-161 |
| Multi-channel micro spectrophotometer | Thermo, USA | Nanodrop 8000 | PD-A1-052 |
| Benchtop refrigerated centrifuge | Thermo, USA | SL16R | PD-A1-082 |
| Clean bench | Airtech, Suzhou | SW-CJ-2FD | QC-A1-011 |
| Medium-flow manual peristaltic pump | Watson Marlow, UK | 520S/R2 | PD-A1-235 |
| Filling machine | Watson Marlow, Denmark | FP50 | PD-C14-115 |
| Insoluble particle detector | Tianda Tianfa, Tianjin | GWJ-8 | QC-A1-094 |

### 1.3. Items and methodology for formulation stability tests

The test items in the whole studies include: (1) the appearance and the presence of visible particles; (2) the protein content in the formulation determined by the ultraviolet method (UV method); (3) the turbidity of the formulation determined by the OD_{350 nm} method; (4) the purity of the antibody formulation determined by size exclusion chromatography-high performance liquid chromatography (SEC-HPLC) and expressed as the percentage of the main peak area to the sum of all peak areas; (5) the purity of the antibody formulation determined by non-reduced capillary electrophoresis-sodium dodecyl sulfate (non-reduced CE-SDS) and expressed as the percentage of the main peak area to the sum of all peak areas; and (6) charge variants in the antibody formulation determined by CEX-HPLC expressed as the percentage of the principal component, acidic component and basic component; (7) relative binding activity of the anti-TIGIT antibody to TIGIT antigen measured by direct ELISA.

### Detection of visible particles

The visible particles in the sample are detected using a clarity detector (model No. YB-2, Tianda Tianfa, Tianjin) and an insoluble particle detector (model No. GWJ-8, Tianda Tianfa, Tianjin) according to the method described in the National Pharmacopoeia Committee, the Pharmacopoeia of the People's Republic of China (2015 edition, volume IV General Rules 0904 "Test for Visible Particles"), Beijing, China Medical Science Press, 2015.

### Determination of protein content

The protein content in the sample is determined using an ultraviolet spectrophotometer (model No. UV-1800, Shimadzu, Japan) and a multi-channel microspectrophotometer (model No. Nanodrop8000, Thermo, USA).

### Determination of turbidity

The turbidity of the sample is determined by measuring the absorbance at 350 nm using an ultraviolet spectrophotometer (model No. UV-1800, Shimadzu, Japan).

### Purity (SEC-HPLC)

The separation is performed on an SEC column using a phosphate buffer (3.12 g of sodium dihydrogen phosphate dihydrate, 8.77 g of sodium chloride and 34.84 g of arginine are dissolved in ultra-pure water, the pH is adjusted to 6.8 by adding hydrochloric acid, and the volume is brought to 1000 mL) as the mobile phase. The chromatographic column protective solution is 0.05% (w/v) NaN₃, the sample injection volume is 50 µL, the flow rate is 0.5 mL/min, the collection time is 30 min, the column temperature is 25 °C, and the detection wavelength is 280 nm. The sample is diluted to 2 mg/mL with ultra-pure water for use as a sample solution. The formulation buffer is diluted in the same manner as described above to prepare a blank solution. The blank solution and the sample solution are separately injected into a liquid chromatograph in an amount of 50 µL for determination.

### Purity (non-reduced CE-SDS)

The determination is conducted by capillary gel electrophoresis. The capillary is an uncoated capillary having an inner diameter of 50 µm, a total length of 30.2 cm and an effective length of 20.2 cm. Before electrophoresis, the capillary column is washed with 0.1 mol/L sodium hydroxide, 0.1 mol/L hydrochloric acid, ultra-pure water, and electrophoresis gel at 70 psi. The sample is diluted to 2.0 mg/mL with an appropriate amount of ultra-pure water. 50 µL of the diluted sample is transferred into a 1.5-mL centrifuge tube, and 45 µL of sample buffer at pH 6.5 (0.32 g of citric acid monohydrate and 2.45 g of disodium phosphate dodecahydrate are dissolved in 45 mL of ultra-pure water, and the volume is brought to 50 mL to prepare a citrate-phosphate buffer; 80 µL of 10% (w/v) sodium dodecyl sulfate solution is added to 200 µL of the buffer, the volume is brought to 1 mL with water, and the mixture is well mixed to give the sample buffer), 1 µL of internal standard (10 kDa protein, 5 mg/mL; Beckman Coulter, Catalog No. 390953) and 5 µL of 250 mmol/L NEM solution (62 mg of *N*-ethylmaleimide is dissolved in 2 mL of ultra-pure water) are added. The mixture is well mixed, heated at 70 ± 2 °C for 10 ± 2 min, cooled to room temperature, and transferred to a sample bottle for future use as a sample solution. The formulation buffer of the same volume as the sample is processed by the same method as above to prepare the blank solution. Conditions for sample injection: -5 kV for 20 s; separation voltage: -15 kV for 35 min. The capillary column temperature is controlled at 25 °C and the detection wavelength is 220 nm.

### Charge variants (CEX-HPLC)

The samples are measured with cation exchange chromatography (CEX-HPLC). The separation is performed on a MabPac SCX-10 strong cation exchange chromatographic column. The mobile phase A is a 10 mmol/L phosphate buffer (0.51 g of NaH₂PO₄·2H₂O and 2.40 g of Na₂HPO₄·12H₂O are dissolved in 800 mL of ultra-pure water, the volume is brought to 1000 mL, and the mixture is filtered through a Φ 0.22 µm filter membrane), and the mobile phase B is a 10 mmol/L phosphate + 200 mmol/L sodium chloride buffer solution (0.51 g of NaH₂PO₄·2H₂O, 2.40 g of Na₂HPO₄·12H₂O and 11.69 g of NaCl are dissolved in 800 mL of ultra-pure water, the volume is brought to 1000 mL, and the mixture is filtered through a Φ 0.22 µm filter membrane). The sample is diluted to 2.0 mg/mL with ultra-pure water as a sample solution. The formulation buffer is diluted in the same manner as described above to prepare a blank solution. The blank solution and the sample solution are separately injected into a liquid chromatograph in an amount of 50 µL for determination. The flow rate of the mobile phase is 1.0 mL/min, the collection time is 35 min, the column temperature is 35 °C, the detection wavelength is 280 nm, and the temperature of the sample tray is 10 °C. The sample solution is injected for analysis, and the contents of the principal component, the acidic component and the basic component are calculated by area normalization.

### Relative binding activity (direct ELISA)

Human TIGIT antigen (purchased from Acrobiosystems, TIT-H52H3) is immobilized on a 96-well ELISA plate at 0.5 µg/mL in a volume of 100 µL/well overnight at 4 °C. After washing, the plate is blocked with a blocking solution (2% BSA-PBST, 300 µL/well) at 37 °C for 2 h. Serially diluted samples are added at 100 µL/well into the plate after the blocking solution is discarded. For negative control wells, only 100 µL of the diluent (2% BSA-PBST) is added. The plate is incubated in a thermostatic incubator at 37 °C for 60 min. After the plate is washed, HRP-conjugated goat anti-human IgG-Fc fragment (BETHYL, USA, Catalog No.: A80-104P) diluted with 2% BSA-PBST is added as the secondary antibody (100000-fold dilution, 100 µL/well) for reaction at 37 °C for 30 min. After the plate is washed, 100 µL of TMB chromogenic solution is added, and after 10 min of chromogenic reaction, 100 µL of 1 mol/L H₂SO₄ is added to each well to terminate the reaction. The OD value at 450 nm is measured with 620 nm being the reference wavelength. By taking concentration values of the sample at all concentration gradients as an abscissa and the OD_{450 nm}-OD_{620 nm} values of the sample at all concentration gradients as an ordinate, EC₅₀ values are calculated by Prism four-parameter fitting to reflect the binding activity of the antibody to each antigen. Relative binding activity (%) = (EC₅₀ of test sample/EC₅₀ of reference sample) × 100%, wherein the reference sample is a stable anti-TIGIT antibody without any stress processing.

### Example 1. Preparation and purification of anti-TIGIT antibody

The anti-TIGIT antibody ADI-30278 that specifically binds to TIGIT was obtained as described in PCT Application No. PCT/CN2019/097665. The antibody has a heavy chain sequence of SEQ ID NO: 9 and a light chain sequence of SEQ ID NO: 10, and is a fully human antibody. PCT Application No. PCT/CN2019/097665 is incorporated herein by reference in its entirety.

Briefly, the antibody was recombinantly expressed in CHO cells and purified by filtration, chromatography, viral inactivation, filtration, and other processes. The samples used in the following pH screening test were products purified by affinity chromatography, having a protein content of 17.0 mg/mL. The samples used in the following formulation determination test were products purified by filtration through a nanofiltration membrane, having a protein content of 11.5 mg/mL.

### Example 2. pH screening test

### 2.1. Procedures

This example examined the effect of histidine buffer systems at pH 5.0, 5.5, 6.0, 6.5 and 7.0 on the stability of the purified anti-TIGIT antibody in Example 1 to give a superior pH range. 10 mM histidine-5% (w/v) sorbitol buffer was prepared, and the pH was adjusted to 5.0, 5.5, 6.0, 6.5 and 7.0 with hydrochloric acid. The purified anti-TIGIT antibody obtained in Example 1 was exchanged into solutions at the different pH by ultrafiltration. The protein content was adjusted to about 20 mg/mL, and polysorbate 80 was added to a concentration of 0.20 mg/mL. The solution was filled into vials, which were then stoppered and capped. The stability of the above samples was examined at 40 ± 2 °C and the specific experimental scheme is shown in Table 1.

**Table 1. Experimental scheme**

| Experimental conditions | Sampling time points | | | Test items |
|---|---|---|---|---|
| | Day 0 | Week 1 | Week 2 | |
| 40°C±2°C | X | X | X | Appearance, visible particles, protein content, turbidity, purity (SEC-HPLC and non-reduced CE-SDS) and charge variants (CEX-HPLC) |

| | | | | |
|---|---|---|---|---|
| Note: (1) X denotes the sampling time point. (2) after sampling at the above time points, the samples were first put into an ultra-low temperature refrigerator and frozen for later detection. | | | | |

### 2.2. Criteria

According to the knowledge of the product and the precision of the instrument and the method, criteria for determining the absence of changes in sample test indexes as compared to initial values were set, so as to determine whether the sample changed, as detailed in Table 2.

**Table 2. Criteria for absence of quality change**

| Test items | Criteria for absence of change |
|---|---|
| Appearance (visual inspection) | Clear to slightly opalescent, colorless to pale yellow liquid free of particles |
| Visible particles (Test for visible particles) | Conforms to the General Rule 0904 of the Pharmacopoeia of the People's Republic of China (2015 edition, volume IV) |
| Protein content (UV method) | Change rate ≤ 10% |
| Turbidity (OD_{350 nm} method) | Change value ≤ 0.02 |
| Purity (SEC-HPLC) | Main peak change ≤ 1% |
| Purity (non-reduced CE-SDS) | Main peak change ≤ 2% |
| Charge variants (CEX-HPLC) | Changes in principal component, acidic component and basic component ≤ 2% |
| Relative binding activity (direct ELISA) | 70%-130% |

### 2.3. Results

### (1) Appearance and visible particles

After the samples at pH 6.5 and pH 7.0 were subjected to concentration and buffer exchange, turbidity and precipitates were found, and the samples were not subjected to further accelerated stability tests. The samples at pH 5.0, pH 5.5 and pH 6.0 were qualified after letting stand for 2 weeks at 40 ± 2 °C for appearance and visible particle.

### (2) Protein content

The results of protein content assay are shown in Table 3. The results showed no significant changes in the protein content for the samples at pH 5.0, pH 5.5 and pH 6.0 after storage at 40 ± 2 °C for 2 weeks.

**Table 3. Protein content results in pH screening test (UV method, mg/mL)**

| Sample name | Time | | |
|---|---|---|---|
| | Day 0 | Week 1 | Week 2 |
| pH 5.0 | 20.1 | 20.0 | 19.9 |
| pH 5.5 | 19.1 | 19.1 | 19.2 |
| pH 6.0 | 20.0 | 20.1 | 20.2 |

### (3) Turbidity

The turbidity results are shown in Table 4. The results showed no changes in turbidity for the samples at pH 5.0 and pH 5.5 and an increase trend in the sample at pH 6.0 after storage at 40 ± 2 °C for 2 weeks.

**Table 4. Turbidity results in pH screening test (OD_{350 nm} method)**

| Sample name | Time | | |
|---|---|---|---|
| | Day 0 | Week 1 | Week 2 |
| pH 5.0 | 0.063 | 0.063 | 0.069 |
| pH 5.5 | 0.074 | 0.075 | 0.075 |
| pH 6.0 | 0.084 | 0.092 | 0.100 |

### (4) Purity

The results of purity (SEC-HPLC and non-reduced CE-SDS) are shown in Table 5. The results showed no significant changes in the purity for the samples at pH 5.0, pH 5.5 and pH 6.0 after storage at 40 ± 2 °C for 2 weeks.

**Table 5. Purity results in pH screening test**

| Test items | Sample name | Time | | |
|---|---|---|---|---|
| | | Day 0 | Week 1 | Week 2 |
| Purity (SEC-HPLC) | pH 5.0 | 97.7 | 98.2 | 98.1 |
| | pH 5.5 | 97.8 | 98.1 | 98.1 |
| | pH 6.0 | 97.9 | 98.0 | 98.0 |
| Purity (non-reduced CE-SDS) | pH 5.0 | 99.1 | 99.0 | 98.8 |
| | pH 5.5 | 99.1 | 99.0 | 98.8 |
| | pH 6.0 | 99.0 | 98.7 | 98.4 |

### (5) Charge variants

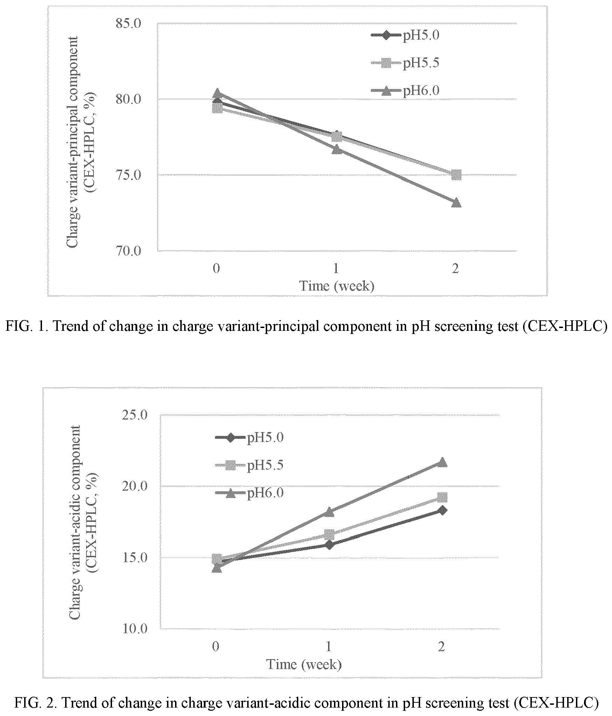

The results of charge variants (CEX-HPLC) are shown in Table 6, and the trends of change are shown in FIGs. 1 and 2. The results showed significant changes in the principal component and acidic component for the samples at pH 5.0, pH 5.5 and pH 6.0 after storage at 40 ± 2 °C for 2 weeks. Compared with day 0, the principal component of the samples at pH 5.0, pH 5.5 and pH 6.0 were decreased by 4.8%, 4.4% and 7.2%, respectively, and the acidic components were increased by 3.6%, 4.3% and 7.4%, respectively. In summary, the antibody was more stable at pH 5.0 and pH 5.5 as indicated by the charge variant results.

**Table 6. Charge variant results in pH screening test (CEX-HPLC, %)**

| Sample name | | Time | | |
|---|---|---|---|---|
| | | Day 0 | Week 1 | Week 2 |
| pH 5.0 | Acidic component | 14.7 | 15.9 | 18.3 |
| | Principal component | 79.8 | 77.6 | 75.0 |
| | Basic component | 5.5 | 6.4 | 6.7 |
| pH 5.5 | Acidic component | 14.9 | 16.6 | 19.2 |
| | Principal component | 79.4 | 77.5 | 75.0 |
| | Basic component | 5.7 | 5.9 | 5.8 |
| pH 6.0 | Acidic component | 14.3 | 18.2 | 21.7 |
| | Principal component | 80.4 | 76.7 | 73.2 |
| | Basic component | 5.3 | 5.1 | 5.1 |

In summary, the pH screening test results indicated that a pH between 5.0 and 5.5 of the histidine buffer system for the antibody formulation is preferable. pH 5.2 was selected for further formulation determination test.

### Example 3. Formulation determination test

### 3.1. Procedures

According to the pH screening test results and experience of the formulation development platform, the effect of different stabilizers (sorbitol, sucrose and arginine hydrochloride) and polysorbate 80 contents on the antibody stability was examined. 3 formulations were designed, and detailed information on formulations is shown in Table 7.

**Table 7. Information on formulations**

| No. | Information on formulation |
|---|---|
| Formulation 1 | histidine at 0.21 mg/mL, histidine hydrochloride at 1.81 mg/mL, sorbitol at 25.00 mg/mL, arginine hydrochloride at 17.91 mg/mL and polysorbate 80 at 0.20 mg/mL, pH 5.2 |
| Formulation 2 | histidine at 0.21 mg/mL, histidine hydrochloride at 1.81 mg/mL, sorbitol at 25.00 mg/mL, arginine hydrochloride at 17.91 mg/mL and polysorbate 80 at 0.50 mg/mL, pH 5.2 |
| Formulation 3 | histidine at 0.21 mg/mL, histidine hydrochloride at 1.81 mg/mL, sucrose at 40.00 mg/mL, arginine hydrochloride at 17.91 mg/mL and polysorbate 80 at 0.20 mg/mL, pH 5.2 |

Buffers of the formulations were prepared according to Table 7, and the solvent of the antibody was replaced with the corresponding formulation solution by ultrafiltration. After buffer exchange, the protein content of the formulations was adjusted to about 50 mg/mL. Polysorbate 80 was added. The solutions were filtered and filled into vials, which were then stoppered and capped. The samples were subjected to stability tests at shaking, freezing-thawing and different temperatures. The specific scheme is shown in Table 8.

**Table 8. Experimental conditions and sampling schedule**

| Name of experiment | Formulation | Experimental conditions and sampling schedule | Test items |
|---|---|---|---|
| Shaking test | Formulation 1 | 650 r/min, room temperature, in the dark; sampling at day 0, day 3 and day 5 In cycles of a 1-day cryopreservation below -30 °C and a following thawing at room temperature; sampling in Cycles 0, 3^{∗} and 6 | Appearance, visible particles, protein content, purity (SEC-HPLC and non-reduced CE-SDS), charge variants (CEX-HPLC) and relative binding activity (direct ELISA) |
| Freezing -thawing test | Formulation 2 | | |
| | Formulation 3 | | |
| 40 °C stability confirmation experiment | Formulation 2 | Standing at 40 °C; sampling at day 0, week 1, week 2 and month 1 | |
| 25 °C stability confirmation experiment | Formulation 2 | Standing at 25 °C; sampling at day 0, month 1 and month 2 | |

| | | | |
|---|---|---|---|
| Note: "^{∗}" indicates that the time point was set for investigation but not tested. | | | |

### 3.2. Criteria

See Table 2 in Example 2 for the specific criteria.

### 3.3. Results

### (1) Shaking test

The results of the shaking test are detailed in Table 9. The results showed that the 3 formulations were qualified for appearance and visible particle after shaking at room temperature in the dark at 650 r/min for 5 days; no significant changes were found for protein content, purity, charge variants and relative binding activity.

**Table 9. Results of shaking test**

| Sample name | Test indexes | | Time (day) | | |
|---|---|---|---|---|---|
| | | | 0 | Day 3 | Day 5 |
| Formulation 1 | Appearance (visual inspection) | | Qualified | Qualified | Qualified |
| | Visible particles (Test for visible particles) | | Qualified | Qualified | Qualified |
| | Protein content (UV method, mg/mL) | | 50.2 | 50.4 | 50.4 |
| | Charge variants (CEX-HPLC, %) | Acidic component | 13.7 | 13.3 | 13.1 |
| | | Principal component | 80.3 | 80.6 | 80.5 |
| | | Basic component | 5.9 | 6.1 | 6.4 |
| | Purity (SEC-HPLC, %) | | 99.1 | 99.1 | 99.1 |
| | Purity (non-reduced CE-SDS, %) | | 98.5 | 98.8 | 98.8 |
| | Relative binding activity (direct ELISA, %) | | 89 | N/A | 93 |
| Formulation 2 | Appearance (visual inspection) | | Qualified | Qualified | Qualified |
| | Visible particles (Test for visible particles) | | Qualified | Qualified | Qualified |
| Sample name | Test indexes | | Time (day) | | |
| | | | 0 | Day 3 | Day 5 |
| | Protein content (UV method, mg/mL) | | 51.1 | 50.9 | 50.6 |
| | Charge variants (CEX-HPLC, %) | Acidic component | 13.7 | 13.4 | 13.3 |
| | | Principal component | 80.3 | 80.5 | 80.4 |
| | | Basic component | 5.9 | 6.1 | 6.3 |
| | Purity (SEC-HPLC, %) | | 99.1 | 99.1 | 99.1 |
| | Purity (non-reduced CE-SDS, %) | | 98.9 | 98.7 | 98.6 |
| | Relative binding activity (direct ELISA, %) | | 91 | N/A | 85 |
| Formulation 3 | Appearance (visual inspection) | | Qualified | Qualified | Qualified |
| | Visible particles (Test for visible particles) | | Qualified | Qualified | Qualified |
| | Protein content (UV method, mg/mL) | | 51.9 | 52.3 | 52.3 |
| | Charge variants (CEX-HPLC, %) | Acidic component | 13.8 | 13.3 | 13.1 |
| | | Principal component | 80.2 | 80.6 | 80.7 |
| | | Basic component | 5.9 | 6.1 | 6.2 |
| | Purity (SEC-HPLC, %) | | 99.1 | 99.1 | 99.1 |
| | Purity (non-reduced CE-SDS, %) | | 98.7 | 98.6 | 98.6 |
| | Relative binding activity (direct ELISA, %) | | 101 | N/A | 96 |

| | | | | | |
|---|---|---|---|---|---|
| Note: N/A indicates that the test item was not set. | | | | | |

### (2) Freezing-thawing test

The results of the freezing-thawing test are detailed in Table 10. The results showed that the 3 formulations were qualified for appearance and visible particle after 6 freezing-thawing repeats; no significant changes were found for protein content, purity, charge variants and relative binding activity.

**Table 10. Results of freezing-thawing test**

| Sample name | Test indexes | | Number of freezing/thawing cycles | |
|---|---|---|---|---|
| | | | 0 | 6 |
| Formulation 1 | Appearance (visual inspection) | | Qualified | Qualified |
| | Visible particles (Test for visible particles) | | Qualified | Qualified |
| | Protein content (UV method, mg/mL) | | 50.2 | 50.6 |
| | Charge variants (CEX-HPLC, %) | Acidic component | 13.7 | 13.8 |
| | | Principal component | 80.3 | 80.4 |
| | | Basic component | 5.9 | 5.8 |
| | Purity (SEC-HPLC, %) | | 99.1 | 99.1 |
| | Purity (non-reduced CE-SDS, %) | | 98.5 | 98.7 |
| | Relative binding activity (direct ELISA, %) | | 89 | 117 |
| Formulation 2 | Appearance (visual inspection) | | Qualified | Qualified |
| | Visible particles (Test for visible particles) | | Qualified | Qualified |
| Sample name | Test indexes | | Number of freezing/thawing cycles | |
| | | | 0 | 6 |
| | Protein content (UV method, mg/mL) | | 51.1 | 50.9 |
| | Charge variants (CEX-HPLC, %) | Acidic component | 13.7 | 13.9 |
| | | Principal component | 80.3 | 80.2 |
| | | Basic component | 5.9 | 5.9 |
| | Purity (SEC-HPLC, %) | | 99.1 | 99.1 |
| | Purity (non-reduced CE-SDS, %) | | 98.9 | 98.8 |
| | Relative binding activity (direct ELISA, %) | | 91 | 90 |
| Formulation 3 | Appearance (visual inspection) | | Qualified | Qualified |
| | Visible particles (Test for visible particles) | | Qualified | Qualified |
| | Protein content (UV method, mg/mL) | | 51.9 | 52.6 |
| | Charge variants (CEX-HPLC, %) | Acidic component | 13.8 | 13.9 |
| | | Principal component | 80.2 | 80.3 |
| | | Basic component | 5.9 | 5.8 |
| | Purity (SEC-HPLC, %) | | 99.1 | 99.1 |
| | Purity (non-reduced CE-SDS, %) | | 98.7 | 98.9 |
| | Relative binding activity (direct ELISA, %) | | 101 | 106 |

The above results showed that no significant difference in the stability of formulations 1, 2 and 3 was found in the shaking and freezing-thawing tests.

Considering that the route of administration for the antibody is intravenous infusion, the low content of polysorbate 80 may affect the protein stability after the sample is diluted by normal saline. In addition, sorbitol and sucrose have no difference in protein protection effect, and if non-reduced sorbitol is selected, the risk of saccharification reaction between excipients during long-term storage can be reduced. Therefore, formulation 2 was selected for stability confirmation experiments at 40 °C and 25 °C.

### (3) 40 °C stability confirmation experiment

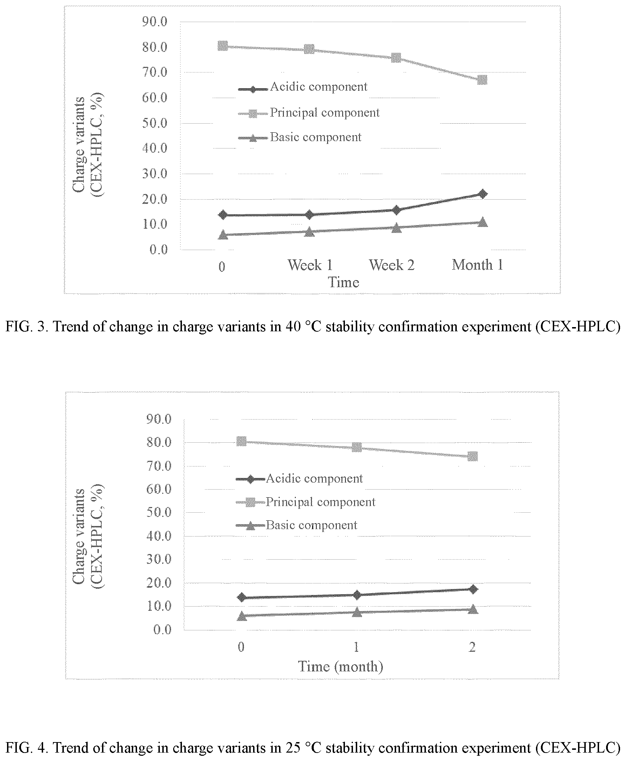

The results of the stability confirmation experiment at 40 °C are detailed in Table 11. After standing for 1 month at 40 °C, the samples were qualified for appearance and visible particles. The protein content, purity and relative binding activity showed no significant changes; the charge variant demonstrated significant changes, with a decrease in principal component by 13.5%, an increase in acidic component by 8.4% and an increase in basic component by 5.1%. The trends of change are shown in FIG. 3. Such changes in charge variants are within the proven acceptable range according to the experience of the formulation development platform.

**Table 11. Results of 40 °C stability confirmation experiment**

| Sample name | Test indexes | | Time | | | |
|---|---|---|---|---|---|---|
| | | | 0 | Week 1 | Week 2 | Month 1 |
| Sample name | Test indexes | | Time | | | |
| | | | 0 | Week 1 | Week 2 | Month 1 |
| Formulation 2 | Appearance (visual inspection) | | Qualified | Qualified | Qualified | Qualified |
| | Visible particles (Test for visible particles) | | Qualified | Qualified | Qualified | Qualified |
| | Protein content (UV method, mg/mL) | | 51.1 | 51.9 | 52.1 | 51.1 |
| | Charge variants (CEX-HPLC, %) | Acidic component | 13.7 | 13.8 | 15.6 | 22.1 |
| | | Principal component | 80.3 | 79.0 | 75.6 | 66.8 |
| | | Basic component | 5.9 | 7.2 | 8.8 | 11.0 |
| | Purity (SEC-HPLC, %) | | 99.1 | 99.1 | 98.8 | 98.2 |
| | Purity (non-reduced CE-SDS, %) | | 98.9 | 98.5 | 98.6 | 98.6 |
| | Relative binding activity (direct ELISA, %) | | 110 | N/A | N/A | 108 |

| | | | | | | |
|---|---|---|---|---|---|---|
| Note: N/A indicates that the test item was not set. | | | | | | |

### (4) 25 °C stability confirmation experiment

The results of the stability confirmation experiment at 25 °C are detailed in Table 12. After standing for 2 months at 25 °C, the samples were qualified for appearance and visible particles. The protein content and purity showed no significant changes; the charge variant demonstrated significant changes, with a decrease in principal component by 6.4%, an increase in acidic component by 3.6% and an increase in basic component by 2.9%. The trends of change are shown in FIG. 5. Such changes in charge variants are within the proven acceptable according to the experience of the formulation development platform.

**Table 12. Results of 25 °C stability confirmation experiment**

| Sample name | Test indexes | | Time | | |
|---|---|---|---|---|---|
| | | | 0 | Month 1 | Month 2 |
| Formulation 2 | Appearance (visual inspection) | | Qualified | Qualified | Qualified |
| | Visible particles (Test for visible particles) | | Qualified | Qualified | Qualified |
| | Protein content (UV method, mg/mL) | | 51.1 | 51.7 | 51.5 |
| | Charge variants (CEX-HPLC, %) | Acidic component | 13.7 | 14.8 | 17.3 |
| | | Principal component | 80.3 | 77.7 | 73.9 |
| | | Basic component | 5.9 | 7.5 | 8.8 |
| | Purity (SEC-HPLC, %) | | 99.1 | 99.0 | 98.9 |
| | Purity (non-reduced CE-SDS, %) | | 98.9 | 99.3 | 98.1 |

According to the above result and the experience of the formulation development platform, formulation 2 was finally selected as the formulation of the antibody. The formulation comprises: the recombinant fully human anti-TIGIT antibody at 50.0 mg/mL, histidine at 0.21 mg/mL, histidine hydrochloride at 1.81 mg/mL, sorbitol at 25.00 mg/mL, arginine hydrochloride at 17.91 mg/mL and polysorbate 80 at 0.50 mg/mL, pH 5.2.

The exemplary embodiments of the present invention have been described above. It should be understood by those skilled in the art that these contents are merely exemplary, and various other replacements, adaptations and modifications can be made within the scope of the present invention. Accordingly, the present invention is not limited to the specific embodiments listed herein.

## Claims

1. A liquid antibody formulation, comprising:
(i) an anti-TIGIT antibody;
(ii) a buffer,
(iii) a stabilizer, and
(iv) a surfactant,
wherein the anti-TIGIT antibody comprises
- a heavy chain VH CDR1 of YTFTEYYMH (SEQ ID NO: 1);
- a heavy chain VH CDR2 of IISPSAGSTKYAQKFQG (SEQ ID NO: 2);
- a heavy chain VH CDR3 of ARDHDIRLAGRLADY (SEQ ID NO: 3);
- a light chain VL CDR1 of RASQGISSWLA (SEQ ID NO: 4);
- a light chain VL CDR2 of AASSLQS (SEQ ID NO: 5); and
- a light chain VL CDR3 of QQAVILPIT (SEQ ID NO: 6),
preferably, the liquid antibody formulation has a pH of about 5.0-6.0, e.g., a pH of 5.2 ± 0.2 or 5.5 ± 0.2, preferably a pH of 5.2.

2. The liquid antibody formulation according to claim 1, wherein the anti-TIGIT antibody in the liquid antibody formulation has a concentration of about 1-100 mg/mL, preferably about 10-70 mg/mL, e.g., about 10, 15, 20, 25, 30, 35, 40, 50, 55, 60 or 70 mg/mL.

3. The liquid antibody formulation according to claim 1 or 2, wherein
the liquid antibody formulation comprises a buffer selected from a histidine-histidine hydrochloride buffer system, a citric acid-sodium citrate buffer system, an acetic acid-sodium acetate buffer system and a phosphate buffer system, and
preferably, the buffer in the liquid antibody formulation is selected from histidine, histidine hydrochloride and a combination thereof;
preferably, the buffer has a concentration of about 5-50 mM, preferably about 5-30 mM, e.g., about 5, 10, 15, 20, 25, 30 mM.

4. The liquid antibody formulation according to any one of claims 1-3, wherein stabilizer is selected from polyols (e.g., sorbitol, mannitol and a combination thereof), saccharides (e.g., sucrose, trehalose, maltose and a combination thereof), amino acids (e.g., arginine, arginine hydrochloride, methionine, glycine, proline and a combination thereof) and any combination thereof,
for example, the stabilizer comprises one or more selected from:
- a polyol selected from sorbitol, mannitol and a combination thereof;
- a saccharide selected from sucrose, trehalose, maltose and a combination thereof;
- an amino acid selected from arginine hydrochloride, methionine, glycine, proline and a combination thereof.

5. The liquid antibody formulation according to any one of claims 1-4, wherein the stabilizer is selected from:
(i) a combination of sorbitol at about 20-40 mg/mL and arginine at about 50-100 mM, and
(ii) a combination of sucrose at about 30-60 mg/mL and arginine at about 50-100 mM.

6. The liquid antibody formulation according to any one of claims 1-4, wherein the surfactant in the liquid antibody formulation is selected from a polysorbate surfactant, poloxamer, polyethylene glycol and a combination thereof, preferably polysorbate 80.

7. The liquid antibody formulation according to any one of claims 1-5, wherein the surfactant has a concentration of about 0.1-1 mg/mL, preferably about 0.2-0.8 mg/mL, e.g., about 0.2, 0.3, 0.4, 0.5, 0.6, 0.7 or 0.8 mg/mL.

8. The liquid antibody formulation according to any one of claims 1-7, wherein the anti-TIGIT antibody comprises a heavy chain variable region VH and a light chain variable region VL, wherein the heavy chain variable region comprises a sequence of SEQ ID NO: 7 or a sequence having at least 90%, 95%, 98% or 99% identity thereto, and the light chain variable region comprises a sequence of SEQ ID NO: 8 or a sequence having at least 90%, 95%, 98% or 99% identity thereto.

9. The liquid antibody formulation according to any one of claims 1-8, wherein the anti-TIGIT antibody is an IgG antibody (e.g., an IgG4 subtype antibody), preferably comprising a heavy chain sequence of SEQ ID NO: 9 or a sequence having at least 90%, 95%, 98% or 99% identity thereto, and a light chain sequence of SEQ ID NO: 10 or a sequence having at least 90%, 95%, 98% or 99% identity thereto.

10. The liquid antibody formulation according to any one of claims 1-9, wherein the anti-TIGIT antibody is recombinantly expressed in 293 cells or CHO cells.

11. The liquid antibody formulation according to any one of claims 1-10, wherein the liquid formulation is an injection, preferably for subcutaneous or intravenous injection, or an infusion, e.g., for intravenous infusion.

12. The liquid antibody formulation according to any one of claims 1-11, wherein the liquid antibody formulation comprises:
(i) an anti-TIGIT antibody at about 10-100 mg/mL;
(ii) a histidine buffer at about 5-50 mM;
(iii) sorbitol or sucrose at about 50-300 mM;
(iv) polysorbate 80 at about 0.1-1 mg/mL; and
(v) optionally, arginine at about 50-120 mM,
wherein the liquid formulation has a pH of about 5.0-5.5, e.g., about 5.2;
for example, the liquid antibody formulation comprises:
(i) an anti-TIGIT antibody at about 10-60 mg/mL, e.g., 20-50 mg/mL;
(ii) a histidine buffer at about 10 mM;
(iii) sorbitol or sucrose at about 10-50 mg/mL, preferably sorbitol at 20-30 mg/mL, or sucrose at 30-50 mg/mL;
(iv) polysorbate 80 at about 0.2-0.8 mg/mL, e.g., 0.3-0.6 mg/mL; and
(v) arginine at about 60-100 mM, e.g., about 85 mM,
wherein the liquid formulation has a pH of about 5.0-5.5, e.g., about 5.2;
or the liquid antibody formulation comprises:
(i) an anti-TIGIT antibody at about 50 mg/mL, histidine at about 0.21 mg/mL, histidine hydrochloride at about 1.81 mg/mL, sorbitol at about 25.00 mg/mL, arginine hydrochloride at about 17.91 mg/mL and polysorbate 80 at about 0.50 mg/mL, about pH 5.2;
(ii) an anti-TIGIT antibody at about 50 mg/mL, histidine at about 0.21 mg/mL, histidine hydrochloride at about 1.81 mg/mL, sorbitol at about 25.00 mg/mL, arginine hydrochloride at about 17.91 mg/mL and polysorbate 80 at about 0.20 mg/mL, about pH 5.2; or
(iii) histidine at 0.21 mg/mL, histidine hydrochloride at 1.81 mg/mL, sucrose at 40.00 mg/mL, arginine hydrochloride at 17.91 mg/mL and polysorbate 80 at 0.20 mg/mL, pH 5.2.

13. The liquid antibody formulation according to any one of claims 1-12, wherein the formulation is stable after storage, e.g., at 25 °C for at least 2 months, or at 40 ± 2 °C for 1 month, and the formulation preferably has one or more of the following characteristics:
(i) a main peak change less than 1%, and/or a purity greater than 96%, preferably greater than 97% or 98% as measured by SEC-HPLC;
(ii) a main peak change less than 2%, and/or a purity greater than 96%, preferably greater than 97% or 98% as measured by non-reduced CE-SDS;
(iii) a sum of change in components (principal component, acidic component and basic component) not more than 40% and/or a change in the principal component not more than 20% of the anti-TIGIT antibody in the formulation relative to an initial value on day 0 of storage as measured by CEX-HPLC, for example, a sum of change not more than about 40% (e.g., not more than 30%) and/or a change in the principal component not more than about 20% (e.g., not more than 15%) after storage at 40 ± 2 °C for 1 month, or a sum of change not more than about 20% (e.g., about 15%) and/or a change in the principal component not more than about 15% (e.g., not more than about 10%) after storage at 25 °C for 2 months; or
(iv) a relative binding activity of the anti-TIGIT antibody in the formulation of 70%-130%, e.g., 90%-110%, relative to an initial value on day 0 of storage as measured by ELISA;
more preferably, the formulation is stable at shaking and/or repeated freezing-thawing.

14. A solid antibody formulation obtained by solidifying the liquid antibody formulation according to any one of claims 1-13, wherein the solid antibody formulation is, e.g., in the form of a lyophilized powder for injection.

15. A delivery device, comprising the liquid antibody formulation according to any one of claims 1-13 or the solid antibody formulation according to claim 14.

16. A pre-filled syringe, comprising the liquid antibody formulation according to any one of claims 1-13 or the solid antibody formulation according to claim 14 for use in intravenous injection or intramuscular injection.

17. Use of the liquid antibody formulation according to any one of claims 1-13 or the solid antibody formulation according to claim 14 in preparing a delivery device, a pre-filled syringe or a medicament that blocks binding of TIGIT to CD155 to reduce or eliminate the immunosuppressive effect of TIGIT in a subject.

18. Use of the liquid antibody formulation according to any one of claims 1-13 or the solid antibody formulation according to claim 14 in preparing a delivery device, a pre-filled syringe or a medicament for treating or preventing a tumor or a pathogen infection in a subject, wherein, for example, the tumor is a cancer in gastrointestinal tract, e.g., colon cancer.

19. The use according to claim 17 or 18, wherein the anti-TIGIT antibody is administered in combination with a second therapeutic agent, for example, an anti-PD-1 antibody.
